# EUROPEAN PATENT APPLICATION

(11) **EP 4 154 883 A1**
(43) Date of publication of application: **29.03.2023**
(21) Application number: 21808653.6
(22) Date of filing: 20.05.2021
(51) Int. Cl.: A61K 31/47, A61P 7/02, A61P 11/00

(54) **DRUG FOR ENHANCING FIBRINOLYTIC SYSTEM, AND USE THEREFOR**

(30) Priority: 20.05.2020 JP 2020088416
(71) Applicant: Renascience Inc., Tokyo 103-0023 (JP)
(72) Inventor: MIYATA, Toshio, Tokyo 103-0023 (JP)
(74) Representative: Vossius & Partner Patentanwälte Rechtsanwälte mbB
(86) International application number: PCT/JP2021/019259
(87) International publication number: WO 2021/235532

(57) **Abstract**

Provided is a drug for enhancing the fibrinolytic system, comprising a compound represented by Formula (1) or a pharmaceutically acceptable salt thereof as an active ingredient: 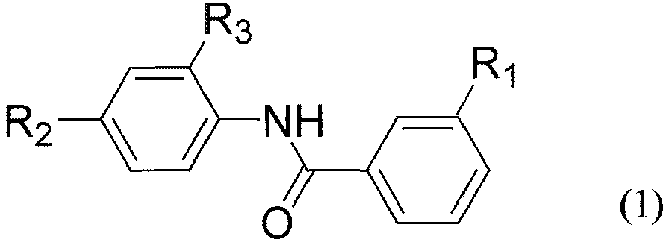 wherein R₁ is quinolyl, R₂ is halogen, and R₃ is carboxyl or a group that is biologically equivalent thereto; the active ingredient being administered at a daily dosage of 120 to 300 mg, and the drug being orally administered to a patient with a disease with a clinical condition that is expected to be improved by fibrinolytic enhancement.

## Description

### Technical Field

The present invention relates to a drug for enhancing the endogenous fibrinolytic system. In particular, the present invention relates to a drug for enhancing the endogenous fibrinolytic system, having an excellent effect of enhancing the fibrinolysis system (hereinafter referred to as "the fibrinolytic system") by endogenous tissue plasminogen activator (hereinafter referred to as "t-PA"), and also having low toxicity and side effects, as well as effectiveness and safety.

### Background Art

Plasminogen activator inhibitor 1 (hereinafter referred to as "PAI-1") is a protein having a function to bind to t-PA, which converts plasminogen to plasmin in the blood fibrinolytic system, to thereby inhibit its action. PAI-1 is synthesized in various parts of the body, such as the liver, vasculature, and visceral fat, and at sites of injury and inflammation, and secreted into plasma. In the fibrinolytic system, when PAI-1 is activated and endogenous t-PA activity is reduced, the production of plasmin, which has a thrombus-dissolving effect (fibrin clotdissolving effect), is suppressed, resulting in the progression of thrombotic tendency. PAI-1 has been reported to be involved in cellular senescence (NPL 1 and NPL 2), and is known to be also involved in hypertension and atherosclerosis (NPL 3 and NPL 4), coronary artery disease (NPL 5 and NPL 6), idiopathic pulmonary fibrosis (IPF) and emphysema (NPL 7 to NPL 9), type 2 diabetes and nonalcoholic steatohepatitis (NPL 10 and NPL 11), and cognitive decline and Alzheimer's disease (NPL 12 and NPL 13), as well as diseases such as alopecia. There is thus a large body of experimental and epidemiological evidence for the association between PAI-1 and diseases associated with aging (aging-related diseases).

Acute respiratory distress syndrome (hereinafter referred to as "ARDS") is acute-onset non-cardiogenic pulmonary edema caused by underlying or secondary disease, such as severe pneumonia or sepsis. Typical pathological features are diffuse alveolar damage (DAD) with neutrophil infiltration into the lung and increased inflammatory cytokines (tumor necrosis factor, IL-1, IL-6, and IL-8). Harmful toxic mediators (reactive oxygen species and proteases) are produced, damaging the capillary endothelium and alveolar epithelium, and causing alveolar edema (NPL 14). Eventually, gas exchange is impaired, pulmonary compliance is reduced, and pulmonary arterial pressure increases (NPL 15). Of the patients infected with the new coronavirus (SARS-CoV-2) (hereinafter, infections caused by this virus are referred to as "COVID-19," based on the official name of the infectious disease "COVID-19"), which was confirmed to occur in Wuhan, China, in December 2019, elderly patients and patients with underlying diseases, such as diabetes, are especially prone to develop ARDS. Many cases have been reported that become more severe and require treatment using ventilators or artificial lungs (ECMO).

Inflammation regulates blood coagulation by enhancing C-reactive protein (CRP) production. CRP stimulates monocytes and alveolar macrophages to generate tissue factor (TF) (NPL 16 and NPL 17), and allows the production of PAI-1 from endothelial cells (NPL 18). The combination of these is considered to cause disseminated intravascular coagulation (DIC), which includes intravascular microthrombosis and formation of fibrin deposits in the alveoli (NPL 17 to NPL 20). This results in both dead space ventilation and intrapulmonary shunting, which are features of ARDS (NPL 19 and NPL 21). There is much evidence linking PAI-1 to the development of ARDS due to pneumonia (NPL 22). Elevated plasma PAI-1 levels are recognized as an independent risk factor for patient mortality and adverse clinical outcomes in patients with acute lung injury or ARDS (NPL 23).

Coronavirus nucleocapsid (N) proteins directly associate with the intracellular signaling molecule Smad3 and enhance PAI-1 expression induced by transforming growth factor-β (TGF-β) (NPL 24). Such regulation of TGF-β signaling by N proteins is considered to inhibit apoptosis of virus-infected host cells and induce tissue fibrosis. In COVID-19 patients, this mechanism may also cause progressive pulmonary fibrosis, and elevated PAI-1 levels may contribute to pathological thrombus formation in the lungs.

Patients with ARDS caused by COVID-19 show not only extensive alveolar damage in the airways, but also the formation of a clear membrane with fibrin components (NPL 25). In addition, diffuse alveolar damage was observed as pathological evidence of pulmonary thrombosis in patients with severe COVID-19. Further, it was reported that fibrinous microthrombi in small pulmonary arteries were observed in 8 out of 10 patients (NPL 26). In a clinical study by Wu et al., 13 COVID-19 patients with moderate to severe lung damage were treated with inhaled plasminogen and showed rapid improvement in lung damage (NPL 27). Zhou et al. also found a significant correlation between high blood d-dimer level (1 ug/mL or more), i.e., hypercoagulability, and mortality in adult patients with COVID-19 in Wuhan, China, suggesting that it is one of the risk factors (NPL 28).

It is essential to promote local fibrinolysis in order to degrade pre-existing fibrin in the lungs. For this reason, the use of t-PA, which is a fibrinolytic agent approved for intravenous thrombolytic therapy, has been proposed for the treatment of ARDS associated with COVID-19. Nebulized t-PA may be an approach to improve oxygen deprivation in COVID-19 patients by degrading fibrin present in the lungs (NPL 29).

As explained above, the fibrinolytic system is involved in ageing-related diseases and ARDS, especially ARDS caused by the current problem of COVID-19; thus, activation of the fibrinolytic system is useful in preventing and treating these diseases and clinical conditions. This suggests that PAI-1 inhibition, which is one of the mechanisms of fibrinolytic system activation, may be a therapeutic tool for ARDS.

On the other hand, many compounds having PAI-1 inhibitory activity have been conventionally known; however, it is not clear whether all of them can be used in clinical practice as PAI-1 inhibitors or drugs for enhancing the fibrinolytic system. In addition, for practical use as clinical drugs, they are required to have not only an excellent effect of enhancing the endogenous fibrinolytic system, but also low toxicity and a wide range of safety. It is also necessary that side effects do not occur even after repeated administration. Furthermore, it is required that they be easily taken. It is also desired that they can be used in combination with other drugs.

### Citation List

### Patent Literature

PTL 1: WO2010/113022

### Non-patent Literature

NPL 1: Kortlever RM, et al., Nature Cell Biology. 2006; 8(8): 877-84.
NPL 2: Ozcan S, et al., Aging (Albany, NY). 2016; 8(7): 1316-29.
NPL 3: Lieb W, et al., Circulation. 2009 Jan 6; 119(1): 37-43.
NPL 4: Boe AE, et al., Circulation. 2013; 128(21): 2318-24.
NPL 5: Song C, et al., J Am Heart Assoc. 2017 May 26; 6(6).
NPL 6: Eren M, et al., Circulation. 2002 Jul 23; 106(4): 491-6.
NPL 7: Eitzman DT et al., J Clin Invest. 1996 Jan 1; 97(1): 232-7. PubMed PMID: 8550840; PubMed Central PMCID: PMC507084.
NPL 8: Rana T et al., Am J Respir Cell Mol Biol 2020; 62: 319-330.
NPL 9: Eren M et al., Proc Natl Acad Sci U S A. 2014; 111(19): 7090-5.
NPL 10: Festa A et al., Diabetes. 2002 Apr; 51(4): 1131-7. PubMed PMID: 11916936.
NPL 11: Campbell PT et al., CARDIA. Liver International 2020.
NPL 12: Gerenu G et al., Biochim Biophys Acta Mol Basis Dis. 2017 Apr; 1863(4): 991-1001.
NPL 13: Jacobsen JS et al., Proc Natl Acad Sci U S A. 2008; 105(25): 8754-8759.
NPL 14: Ware LB et al., N Engl J Med. 2000; 342: 1334-49.
NPL 15: Rawal G et al., J Transl Int Med. 2018; 6: 74-77.
NPL 16: Idell S et al., Am J Physiol. 1991; 261: L240-8.
NPL 17: Xue M et al., J Transl Med (2015) 13: 172.
NPL 18: Prabhakaran P et al., Am J Physiol Lung Cell Mol Physiol (2003) 285(1): L20-8.
NPL 19: Brun-Buisson C et al., Intensive Care Med (2004) 30(1): 51-61.
NPL 20: Sapru A et al., Intensive Care Med (2010) 36(1): 157-163.
NPL 21: Ozolina et al. A Prospective Pilot Study Front Med (Lausanne) 2016; 3: 64. Published online 2016 Nov 28.
NPL 22: El Solh AA et al., Intensive Care Med. 2006; 32(1): 110-115.
NPL 23: Ware LB et al., Crit Care Med. 2007; 35(8): 1821-1828.
NPL 24: Zhao X et al., J Biol Chem. 2008; 283(6): 3272-3280.
NPL 25: Zhe Xu et al., Lancet Respir Med 2020; 8: 420-422.
NPL 26: Dolhnikoff et al., J Thromb Haemost. 2020; 18: 1517-19.
NPL 27: Wu Y et al., QJM. 2020; 113: 539-45.
NPL 28: Zhou F et al., Lancet. 2020; 395: 1054-62.
NPL 29: Whyte CS et al. J Thromb Haemost. 2020; 18: 1548-55.
NPL 30: Bhandary et al. 2015. Plos One 10: e0123187.
NPL 31: Boe et al. 2015. Plos One. 10 e0116504.
NPL 32: Huang WT et al. Am J Respir Cell Mol Biol. 2012 Jan; 46(1): 87.
NPL 33: Liu RM et al. 2016. Am J Physiol Lung Cell Mol Physiol. 310: L328.

### Summary of Invention

### Technical Problem

An object of the present invention is to provide a drug for enhancing the fibrinolytic system that has excellent effects and few side effects, is easy to take, and can be used in combination with other drugs. Another object of the present invention is to provide suitable indications (use) of the drug for enhancing the fibrinolytic system.

### Solution to Problem

The present inventors have been working on the elucidation of the physiological action of PAI-1 and the development of small-molecule PAI-1 inhibitors for many years. A compound represented by Formula (1) or a pharmaceutically acceptable salt thereof (hereinafter these are collectively referred to as "the present compound"), described later, was newly created by the present inventors as a compound having PAI-1 inhibitory activity (PAI-1 inhibitor) (PTL 1).
t-PA secreted from vascular endothelial cells remains in endothelial cells to exert efficient fibrinolytic activity. In contrast, PAI-1 detaches t-PA from the endothelium to form a complex. The present compound selectively and specifically binds to PAI-1 to thereby suppress the binding of PAI-1 to t-PA, and enhance the fibrinolytic system by t-PA. That is, the present compound has the effect of specifically dissolving preformed intravascular thrombi by activating the fibrinolytic system via t-PA. Therefore, its mechanism of action is different from that of existing antithrombotic drugs inhibiting the process of thrombus formation, and is considered to have few hemorrhagic side effects. In addition, the risk of bleeding can be avoided by reducing the dose of t-PA, which is a type of fibrinolytic therapy drug, when used in combination.

The present inventors carried out detailed studies on the stability, effectiveness, and safety of the present compound, which has such a fibrinolytic enhancing effect due to PAI-1 inhibition, by conducting an accelerated stability study, *in vitro* study, *in vivo* studies using animals (non-clinical studies), and clinical studies on humans. As a result, it was confirmed that the present compound was stable for at least 6 months in the accelerated stability study, that there was no toxicity (general toxicity, genotoxicity, reproductive and embryonic developmental toxicity, central, cardiovascular, and respiratory system toxicity, etc.) in the non-human animal studies, that oral administration of the present compound at a daily dosage rate of 120 mg to adult patients had a significant effect, and that no adverse events were observed with single or multiple doses at a rate of 120 mg to 240 mg. It was also confirmed that the present compound can be taken simultaneously with other drugs. From these, it is considered that the present compound can be effectively used as an effective PAI-1 inhibitor with few side effects, particularly as a drug for enhancing the endogenous fibrinolytic system, for the prevention or treatment of diseases in which fibrinolytic enhancement contributes to improvement of clinical conditions, specifically diseases with thrombotic tendency (e.g., thrombotic diseases (e.g., myocardial infarction, cerebral infarction, venous thrombosis, pulmonary thrombosis, and intrapulmonary microthrombosis), and ARDS (including ARDS caused by COVID-19)), fibrosis, emphysema, inflammatory diseases, and clinical conditions thereof.

The present invention has been completed as a result of further research based on these findings, and the invention encompasses the following embodiments.

### (I) Drug for enhancing endogenous fibrinolytic system

(I-1) A drug for enhancing the fibrinolytic system, comprising a compound represented by Formula (1) or a pharmaceutically acceptable salt thereof as an active ingredient: 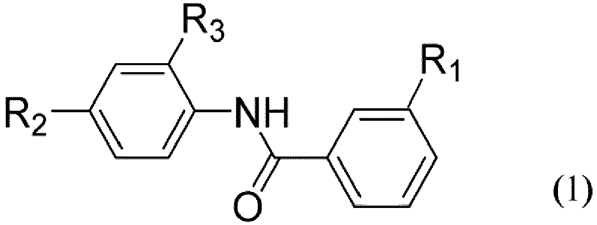 wherein R₁ is quinolyl, R₂ is halogen, and R₃ is carboxyl or a group that is biologically equivalent thereto;
   the active ingredient being administered at a daily dosage of 120 to 300 mg, and
   the drug being orally administered to a patient with a clinical condition that is expected to be improved by fibrinolytic enhancement, in other words, a patient with a disease in which fibrinolytic enhancement contributes to the improvement of the clinical condition, preferably an adult patient aged 15 years or older.
(I-2) The drug for enhancing the fibrinolytic system according to (1-1), wherein the compound is a compound of Formula (1) wherein R₁ is quinolin-8-yl and R₃ is carboxyl, preferably 5-chloro-2-({[3-(quinolin-8-yl)phenyl]carbonyl}amino)benzoic acid.
(I-3) The drug for enhancing the fibrinolytic system according to (I-1) or (I-2), wherein the daily dosage of the active ingredient is 120 to 240 mg.
(I-4) The drug for enhancing the fibrinolytic system according to any one of (I-1) to (I-3), which is repeatedly administered orally once daily for at least 7 days.
(I-5) The drug for enhancing the fibrinolytic system according to any one of (I-1) to (I-4), wherein the patient is a patient having thrombosis, fibrosis, emphysema, and/or inflammation in a respiratory organ.
(I-6) The drug for enhancing the fibrinolytic system according to any one of (I-1) to (I-5), for use in prevention or treatment of at least one disease or clinical condition selected from the group consisting of intrapulmonary microthrombosis, pulmonary fibrosis, emphysema, and lung inflammation.
(I-7) The drug for enhancing the fibrinolytic system according to any one of (I-1) to (I-6), wherein the patient is a pneumonia patient with a coronavirus infection.

That is, the present invention can be regarded as a preventive or therapeutic drug for intrapulmonary microthrombosis, pulmonary fibrosis, emphysema, and/or inflammation of respiratory organs, including the lungs, comprising the compound represented by Formula (1) or a pharmaceutically acceptable salt thereof as an active ingredient. The preventive or therapeutic drug includes pharmaceutical compositions for use in improvement of these diseases or clinical conditions of patients with the diseases or clinical conditions (including pneumonia patients with coronavirus infections) or suppression of aggravation, and for use in suppression of progression to acute respiratory distress syndrome (ARDS).

### (II) Method for improving clinical condition or suppressing aggravation by fibrinolytic enhancement

(II-1) A method for improving a clinical condition that is expected to be improved by fibrinolytic enhancement or suppressing the aggravation of the clinical condition, the method comprising orally administering a pharmaceutical composition comprising a compound represented by Formula (1) or a pharmaceutically acceptable salt thereof as an active ingredient: 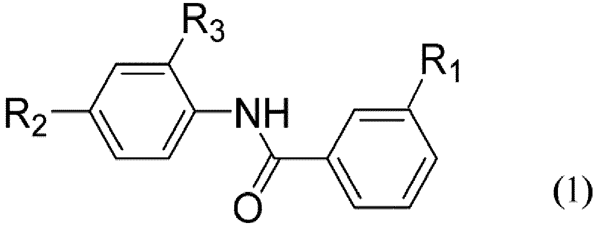 wherein R₁ is quinolyl, R₂ is halogen, and R₃ is carboxyl or a group that is biologically equivalent thereto; to a patient with the clinical condition, preferably an adult patient aged 15 years or older,
   the pharmaceutical composition being orally administered at a rate such that the daily dosage for the patient is 120 to 300 mg, preferably 120 to 240 mg, in terms of the amount of compound (1).
(II-2) The method according to (II-1), wherein the compound is a compound of Formula (1) wherein R₁ is quinolin-8-yl and R₃ is carboxyl, preferably 5-chloro-2-({[3-(quinolin-8-yl)phenyl]carbonyl}amino)benzoic acid.
(II-3) The method according to (II-1) or (II-2), wherein the pharmaceutical composition is repeatedly administered orally once daily for at least 7 days.
(II-4) The method according to any one of (II-1) to (II-3), wherein the patient is a patient having thrombosis, fibrosis, emphysema, and/or inflammation in a respiratory organ.
(II-5) The method according to (II-4), wherein the patient is a patient having at least one clinical condition selected from the group consisting of intrapulmonary microthrombosis, pulmonary fibrosis, emphysema, and lung inflammation.
(II-6) The method according to any one of (II-1) to (11-5), wherein the patient is a pneumonia patient with a coronavirus infection.
(II-7) The method according to (II-5) or (II-6), wherein the suppression of aggravation is to suppress the progression of intrapulmonary microthrombosis, pulmonary fibrosis, emphysema, and/or lung inflammation to ARDS.

### (III) Pharmaceutical composition for use in fibrinolytic enhancement

(III-1) A pharmaceutical composition for use in a method for improving a clinical condition that is expected to be improved by fibrinolytic enhancement or suppressing the aggravation of the clinical condition for a patient with the clinical condition,
   the pharmaceutical composition comprising a compound represented by Formula (1) or a pharmaceutically acceptable salt thereof as an active ingredient: 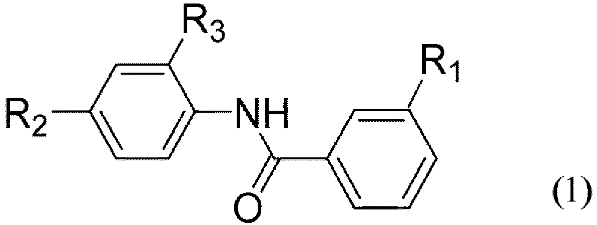 wherein R₁ is quinolyl, R₂ is halogen, and R₃ is carboxyl or a group that is biologically equivalent thereto;
   the method comprising orally administering the pharmaceutical composition to the patient at a rate such that the daily dosage of compound (1) is 120 to 300 mg, preferably 120 to 240 mg.
(III-2) The pharmaceutical composition according to (III-1), wherein the compound is a compound of Formula (1) wherein R₁ is quinolin-8-yl and R₃ is carboxyl, preferably 5-chloro-2-({[3-(quinolin-8-yl)phenyl]carbonyl}amino)benzoic acid.
(III-3) The pharmaceutical composition according to (III-1) or (III-2), wherein the method comprises repeatedly administering orally the pharmaceutical composition to the patient once daily for at least 7 days.
(III-4) The pharmaceutical composition according to any one of (III-1) to (III-3), wherein the patient is a patient having thrombosis, fibrosis, emphysema, and/or inflammation in a respiratory organ.
(III-5) The pharmaceutical composition according to any one of (III-1) to (III-4), wherein the patient is a patient having at least one clinical condition selected from the group consisting of intrapulmonary microthrombosis, pulmonary fibrosis, emphysema, and lung inflammation.
(III-6) The pharmaceutical composition according to any one of (III-1) to (III-5), wherein the patient is a pneumonia patient with a coronavirus infection.
(III-7) The pharmaceutical composition according to (III-5) or (III-6), wherein the method for suppressing the aggravation of the clinical condition is to suppress the progression of intrapulmonary microthrombosis, pulmonary fibrosis, emphysema, and/or lung inflammation to ARDS.

### (IV) Use of pharmaceutical composition for producing drug for enhancing fibrinolytic system

(IV-1) Use of a compound represented by Formula (1) or a pharmaceutically acceptable salt thereof for producing a drug for enhancing the fibrinolytic system: 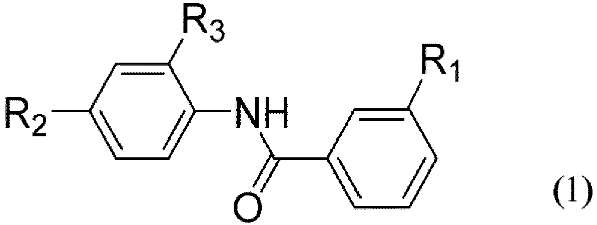 wherein R₁ is quinolyl, R₂ is halogen, and R₃ is carboxyl or a group that is biologically equivalent thereto;
   the drug for enhancing the fibrinolytic system being orally administered at a daily rate of 120 to 300 mg, preferably 120 to 240 mg, to a patient with a clinical condition that is expected to be improved by fibrinolytic enhancement.
(IV-2) The use according to (IV-1), wherein the compound is a compound of Formula (1) wherein R₁ is quinolin-8-yl and R₃ is carboxyl, preferably 5-chloro-2-({[3-(quinolin-8-yl)phenyl]carbonyl}amino)benzoic acid.
(IV-3) The use according to (IV-1) or (IV-2), wherein the drug for enhancing the fibrinolytic system is repeatedly administered orally once daily for at least 7 days.
(IV-4) The use according to any one of (IV-1) to (IV-3), wherein the patient is a patient having thrombosis, fibrosis, emphysema, and/or inflammation in a respiratory organ.
(IV-5) The use according to any one of (IV-1) to (IV-4), wherein the patient is a patient having at least one disease or clinical condition selected from the group consisting of intrapulmonary microthrombosis, pulmonary fibrosis, emphysema, and lung inflammation.
(IV-6) The use according to any one of (IV-1) to (IV-5), wherein the patient is a pneumonia patient with a coronavirus infection.
(IV-7) The use according to (IV-5) or (IV-6), wherein the aggravation suppressing method is to suppress the progression of intrapulmonary microthrombosis, pulmonary fibrosis, emphysema, and/or lung inflammation to ARDS.

### Advantageous Effects of Invention

The drug for enhancing the fibrinolytic system comprising the compound represented by Formula (1) or a pharmaceutically acceptable salt thereof as an active ingredient is orally administered such that the daily dosage of the active ingredient is 120 to 340 mg, preferably 120 to 240 mg, to inhibit PAI-1 and activate t-PA, thereby significantly enhancing the patient's fibrinolytic system without causing problematic side effects. That is, the drug for enhancing the fibrinolytic system of the present invention has the characteristic of being orally administered at the above dosage, and thereby exhibits beneficial effects while ensuring high safety. For these reasons, it is a highly useful drug.

Further, the drug for enhancing the fibrinolytic system of the present invention has an inhibitory effect on intrapulmonary microthrombosis, and further has, based on its PAI-1 inhibition mechanism, a pulmonary fibrosis suppressive effect, an emphysema suppressive effect, an inflammation (cytokine storm)-improving effect, and/or a protective effect on lung epithelial cells. Therefore, it is useful as a practical medicine for preventing the aggravation of respiratory inflammation associated with coronavirus infections, including COVID-19, and progression to ARDS.

### Brief Description of Drawings

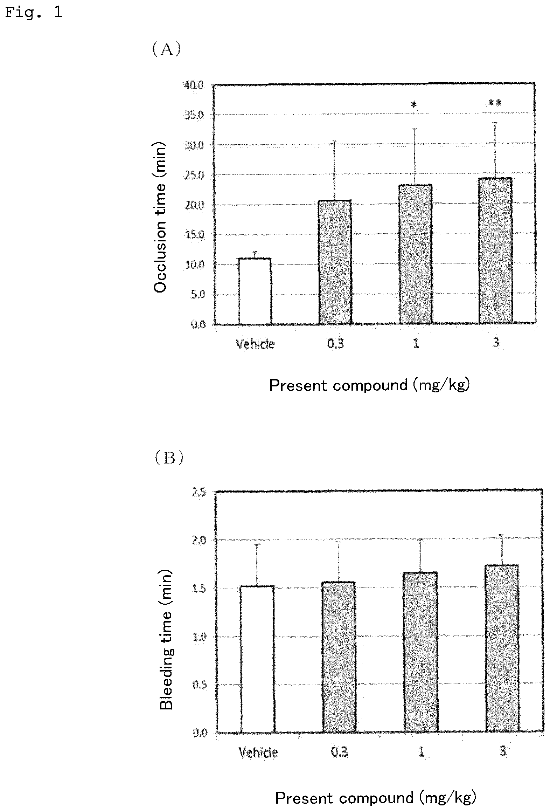

Fig. 1 shows the evaluation results of the antithrombotic effect of the present compound on a rat model of ferric chloride-induced carotid thrombosis in (1) of Experimental Example 3. Fig. 1(A) shows the measurement results of the occlusion time, and Fig. 1(B) shows the measurement results of the bleeding time. In Fig. 1(A), "*" and "**" indicate significant differences (*: P<0.05, **: P<0.01) from the vehicle group.

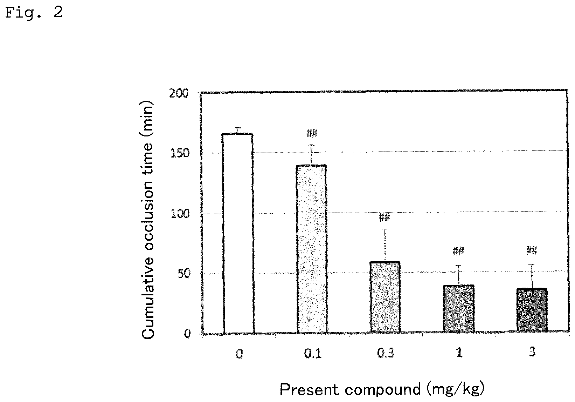

Fig. 2 shows the evaluation results of the antithrombotic effect of the present compound on a cynomolgus monkey model of saphenous arterial thrombus in (2) of Experimental Example 3. Fig. 2 shows the measurement results of the cumulative occlusion time. In Fig. 2, "##" indicates a significant difference (P<0.05) from the vehicle group (0 mg/kg).

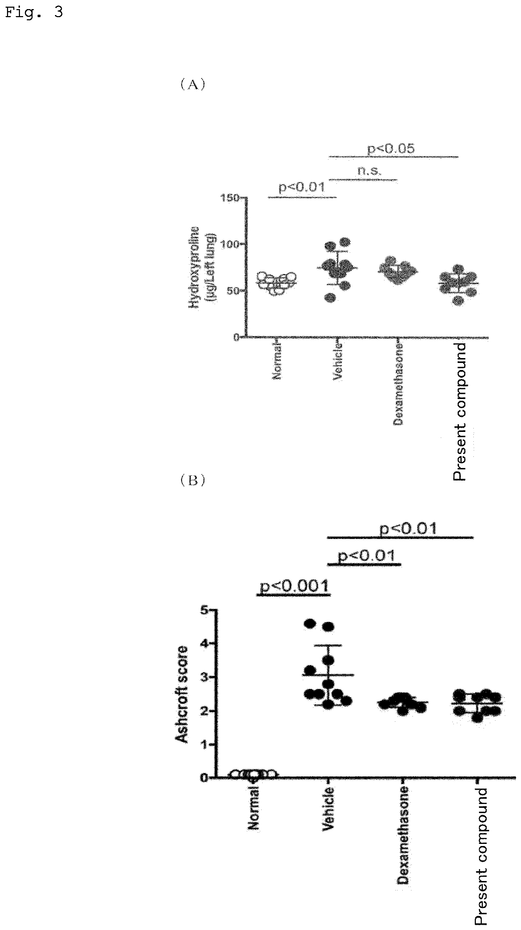

Fig. 3 shows the evaluation results of the pulmonary fibrosis-improving effect of the present compound on a mouse model of bleomycin-induced pulmonary fibrosis in Experimental Example 4. Fig. 3(A) shows the results of comparing the lung hydroxyproline values of the normal group, the vehicle group, the dexamethasone administration group, and the present compound administration group. Fig. 3(B) is the results of comparing the Ashcroft scores calculated from the results of a histopathological test (Masson's trichrome staining) of the above four groups.

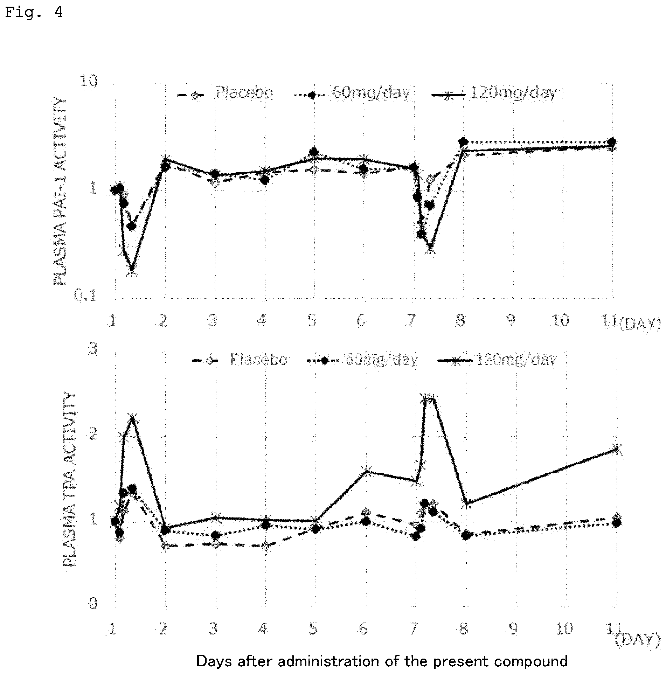

Fig. 4 shows the measurement results of PAI-1 activity and t-PA activity in blood before and after oral administration of the present compound (60 mg/day and 120 mg/day) to humans in the clinical study (phase I study) of Experimental Example 8.

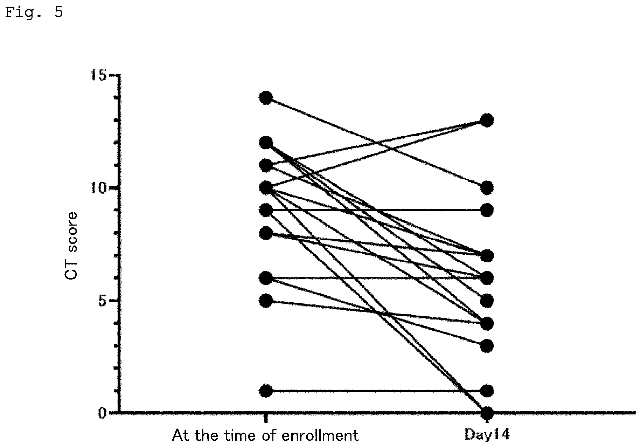

Fig. 5 shows the results of observed changes in the proportion of lesions in the lung field on chest CT images before and after oral administration of the present compound (120 mg/day to 180 mg/day) to patients with COVID-19 pneumonia (mild to moderate) in the effectiveness study for COVID-19 pneumonia (exploratory early phase II study) of Experimental Example 10. Paired t-test, p = 0.0018.

### Description of Embodiments

The present invention relates to a drug for enhancing the fibrinolytic system, comprising a compound represented by Formula (1) or a pharmaceutically acceptable salt thereof (hereinafter these are also referred to collectively as "the present compound") as an active ingredient:

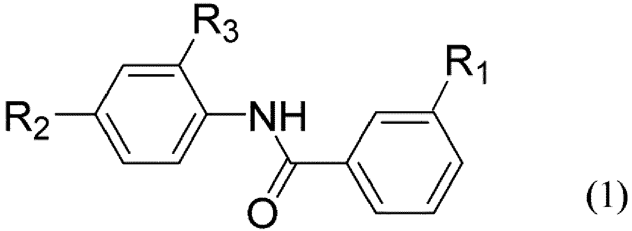

### (1) Present Compound

In Formula (1), R₁ is quinolyl.

Examples of the quinolyl include unsubstituted quinolyl and quinolyl having one or two substituents at any position. Such substituents can be suitably selected as long as the effects of the present invention are not impaired. Examples include halogen, alkyl, halogenated alkyl, alkoxy, halogenated alkoxy, hydroxyl, CF₃, CF₃O, CHF₂O, CF₃CH₂O, phenyl, halogenated phenyl, cyano, carboxyl, alkoxycarbonyl, and the like.

Examples of the halogen in "halogen," "halogenated alkyl," "halogenated alkoxy," and "halogenated phenyl" include chlorine, fluorine, bromine, and iodine. Preferable are chlorine and fluorine.

Examples of the alkyl in "alkyl" and "halogenated alkyl" include C₁₋₄ linear or branched alkyl groups; specifically methyl, ethyl, propyl, isopropyl, n-butyl, isobutyl, sec-butyl, and tert-butyl.

Examples of the alkoxy in "alkoxy," "halogenated alkoxy," and "alkoxycarbonyl" include C₁₋₄ linear or branched alkoxy groups; specifically methoxy, ethoxy, 1-propoxy, 2-propoxy, 1-butoxy, 2-butoxy, 2-methyl-1-propoxy, and 2-methyl-2-propoxy.

Preferable is unsubstituted quinolyl.

The binding site to the adjacent benzene ring is not limited as long as the effects of the present invention are not impaired. Examples include position 8 (quinolin-8-yl), position 6 (quinolin-6-yl), and position 3 (quinolin-3-yl); preferable is position 8.

In Formula (1), R₂ is halogen.

Examples of the halogen include chlorine, fluorine, bromine, and iodine. Preferable are chlorine and fluorine; chlorine is more preferable.

In Formula (1), R₃ is carboxyl or a group that is biologically equivalent thereto.

The group that is biologically equivalent to carboxyl includes the following groups:
(a) groups that are converted to carboxyl when absorbed *in vivo;* and
(b) groups that are known to be biologically equivalent to carboxyl.

Groups (a) include, but are not limited to, COOR₄. In COOR₄, R₄ is alkyl, phenyl, benzyl, (5-C₁₋₆ alkyl-2-oxo-1,3-dioxolen-4-yl)methyl, -CH (R₅)-O-COR₆, or CH(R₅)-O-CO-OR₆ (R₅ is hydrogen or C₁₋₆ alkyl, and R₆ is C₁₋₆ alkyl or C₃₋₈ cycloalkyl) . Preferable among groups (a) is alkoxycarbonyl wherein R₄ is alkyl. Examples of the alkoxycarbonyl include alkoxycarbonyl groups wherein R₄ is C₁₋₆ alkyl, such as t-butoxycarbonyl, methoxycarbonyl, ethoxycarbonyl, propoxycarbonyl, isopropoxycarbonyl, and butoxycarbonyl.

Examples of groups (b) include, but are not limited to, heterocyclic groups such as 1H-tetrazol-5-yl, 4,5-dihydro-5-oxo-4H-1,2,4-oxadiazol-3-yl, 4,5-dihydro-5-oxo-1,2,4-thiadiazol-3-yl, and 4,5-dihydro-5-thioxo-4H-1,2,4-oxadiazol-3-yl (see, for example, Kohara et al., J. Med. Chem., 1996, 39, 5228-5235).

Preferable examples of the compound represented by Formula (1) (compound (1)) include 5-chloro-2-({[3-(quinolin-8-yl)phenyl]carbonyl}amino)benzoic acid and bioisosteres thereof (compounds of Formula (1), wherein R₃ is a group that is biologically equivalent to carboxyl). The compound represented by Formula (1), including this compound, can be produced based on the disclosure of PTL 1, mentioned above.

The present compound may be in free or salt form.

Examples of salts as used herein typically include pharmaceutically acceptable salts, e.g., a salt formed with an inorganic base or organic base, a salt formed with a basic amino acid, and other salts. Examples of inorganic bases include alkali metals such as sodium, potassium, etc.; alkaline earth metals such as calcium, magnesium, etc.; and aluminum, ammonium, etc. Examples of organic bases include primary amines such as ethanolamine, tromethamine, ethylenediamine, etc.; secondary amines such as diethylamine, diethanolamine, meglumine, dicyclohexylamine, N,N'-dibenzylethylenediamine, etc.; and tertiary amines such as trimethylamine, triethylamine, pyridine, picoline, triethanolamine, etc. Examples of basic amino acids include arginine, lysine, ornithine, histidine, etc. Preferably, salts with alkali metals such as sodium can be used. Further, the present compound may form a salt with an inorganic acid or organic acid. Examples of inorganic acids include hydrochloric acid, hydrobromic acid, sulfuric acid, phosphoric acid, etc. Examples of organic acids include formic acid, acetic acid, trifluoroacetic acid, maleic acid, tartaric acid, fumaric acid, citric acid, lactic acid, methanesulfonic acid, benzenesulfonic acid, toluenesulfonic acid, etc.

The present compound targeted by the present invention includes those having a solvate form. Further, so-called prodrugs, which are metabolized *in vivo* to form compounds represented by Formula (I), are also included in the range of the present compound.

### (2) Drug for Enhancing Fibrinolytic System

The present compound can be prepared as a pharmaceutical composition by mixing a pharmaceutically acceptable carrier and additive suitable for its dosage form. The pharmaceutical composition of the present invention contains an effective amount of the present compound to thereby exhibit a PAI-1 inhibitory effect. As a result, the pharmaceutical composition of the present invention has an effect of activating t-PA to increase fibrin degradation by plasmin and enhance the fibrinolytic system *in vivo.* The effective amount may be within the range in which the above effect is exhibited, and can be suitably set within a range of 0.1 to 100 mass%. Therefore, the pharmaceutical composition of the present invention can be preferably used as a drug for enhancing the fibrinolytic system. Specifically, the pharmaceutical composition of the present invention is useful as a preventive or therapeutic drug for PAI-1 activity-related diseases and their clinical conditions (including symptoms; the same below), and particularly for diseases and clinical conditions that will be improved by fibrinolytic enhancement.

In the present invention, the term "prevention" includes the meaning of suppressing the onset of a disease or its clinical condition. Further, in the present invention, the term "treatment" includes the meaning of improving, alleviating, and/or curing a disease or its clinical condition, as well as the meaning of suppressing the progression (including aggravation due to exacerbation) of the disease or its clinical condition.

The diseases and their clinical conditions on which the drug for enhancing the fibrinolytic system of the present invention effectively acts include thrombotic diseases and clinical conditions. Examples include, but are not limited to, various diseases or clinical conditions associated with thrombus formation, such as intravascular microthrombosis (e.g., intrapulmonary microthrombosis), disseminated intravascular coagulation (DIC), venous thrombosis (e.g., deep-vein thrombosis in extremities and pulmonary embolism), arterial thrombosis (e.g., cerebral thrombosis, cerebral infarction, and transient ischemic disease), disorders associated with diabetic complications (e.g., angiopathy, neuropathy, nephropathy, and retinopathy), deep-vein thrombosis (DVT) during surgery, and restenosis after percutaneous transluminal coronary angioplasty (PTCA) .

The diseases and their clinical conditions on which the drug for enhancing the fibrinolytic system of the present invention effectively acts also include diseases and their clinical conditions related to tissue or organ fibrosis, based on the PAI-1 inhibitory effect of the present compound. Examples of such diseases and their clinical conditions include, but are not limited to, pulmonary fibrosis, tissue fibrosis associated with myocardial infarction, and tissue fibrosis associated with nephropathy. Further, the diseases and their clinical conditions on which the drug for enhancing the fibrinolytic system of the present invention effectively acts also include emphysema, and various inflammations and their clinical conditions (e.g. various clinical conditions resulting from the release of inflammatory cytokines (including, for example, cytokine release syndrome and cytokine storm)), based on the PAI-1 inhibitory effect of the present compound. The drug for enhancing the fibrinolytic system of the present invention can be effectively used to improve these diseases and their clinical conditions and prevent aggravation, and particularly to prevent the progression of lung inflammation caused by coronavirus infections to ARDS.

Hereinafter, the drug for enhancing the fibrinolytic system of the present invention is also collectively referred to as "the pharmaceutical composition of the present invention," meaning that it is a pharmaceutical preparation that can be used for the various applications described above.

The pharmaceutical composition of the present invention has an orally administered dosage form. Examples of the orally administered dosage form include, but are not limited to, powders, granules, capsules, pills, tablets, masticatories, sublingual agents, buccal tablets, troches, suspensions, emulsions, and syrups. The dosage form can be suitably selected from these. Such preparations can be imparted with sustainedrelease properties, stabilization, easy degradation, difficult degradation, enteric properties, easy-adsorption properties, etc.

The pharmaceutical composition of the present invention can contain a pharmaceutically acceptable carrier and additive according to the dosage form. Examples of pharmaceutically acceptable carriers and additives include solvents, excipients, binders, lubricants, disintegrators, solubilizers, suspending agents, thickening agents, emulsifiers, stabilizers, buffers, corrigents, coloring agents, coating agents, and the like. Specific examples of pharmaceutically acceptable carriers and additives are mentioned below; however, the present invention is not limited thereto.

Solvents: purified water, sterile purified water, water for injection, physiologic saline, peanut oil, ethanol, glycerol, etc.

Excipients: starches (e.g., potato starch, wheat starch, and corn starch), lactose, dextrose, saccharose, crystalline cellulose, calcium sulfate, calcium carbonate, sodium hydrogencarbonate, sodium chloride, talc, titanium oxide, trehalose, xylitol, etc.

Binders: starch and starch derivatives, cellulose and cellulose derivatives (e.g., methylcellulose, ethylcellulose, hydroxypropylcellulose, and carboxymethylcellulose), natural high-molecular-weight compounds, such as gelatin, sodium arginine, tragacanth, gum arabic, etc., synthetic high-molecular-weight compounds, such as polyvinyl pyrrolidone, polyvinyl alcohol, etc., dextrin, hydroxypropyl starch, and the like.

Lubricants: light anhydrous silicic acid, stearin acid and salts thereof (e.g., magnesium stearate), talc, waxes, wheat starch, macrogol, hydrogenated vegetable oil, sucrose fatty acid ester, polyethylene glycol, silicone oil, etc.

Disintegrators: starch and starch derivatives, agar, gelatin powder, sodium hydrogencarbonate, calcium carbonate, cellulose and cellulose derivatives, hydroxypropyl starch, carboxymethylcellulose, salts thereof, and bridging materials thereof, low-substituted hydroxypropylcellulose, etc.

Solubilizers: cyclodextrin, ethanol, propylene glycol, polyethylene glycol, etc.

Suspending agents: sodium carboxymethylcellulose, polyvinylpyrrolidone, gum arabic, tragacanth, sodium arginine, aluminum monostearate, citric acid, various surfactants, etc.

Thickening agents: sodium carboxymethylcellulose, polyvinylpyrrolidone, methylcellulose, hydroxypropyl methylcellulose, polyvinyl alcohol, tragacanth, gum arabic, sodium arginine, etc.

Emulsifiers: gum arabic, cholesterol, tragacanth, methylcellulose, lecithin, various surfactants (e.g., polyoxyl 40 stearate, sorbitan sesquioleate, polysorbate 80, and sodium lauryl sulfate), etc.

Stabilizers: tocopherol, chelating agents (e.g., EDTA and thioglycolic acid), inert gases (e.g., nitrogen and carbon dioxide), reducing substances (e.g., sodium hydrogen sulfite, sodium thiosulfate, ascorbic acid, and rongalite), etc.

Buffers: sodium hydrogenphosphate, sodium acetate, sodium citrate, boric acid, etc.

Corrigents: saccharose, saccharin, *Glycyrrhiza* extract, sorbitol, xylitol, glycerol, etc.

Coloring agents: water-soluble food colors, lake pigment, etc.

Coating agents: saccharose, hydroxypropylcellulose (HPC), shellac, gelatin, glycerol, sorbitol, hydroxypropyl methylcellulose (HPMC), ethylcellulose, polyvinyl pyrrolidone (PVP), hydroxypropylmethylcellulose phthalate (HPMCP), cellulose acetate phthalate (CAP), methyl methacrylate-methacrylic acid copolymer, polymers described above, etc.

Known drug delivery systems (DDS) can be applied for the dosage forms given above. The term "DDS preparation" as used in the present specification refers to slow-release preparations, locally applied preparations (troches, buccal tablets, sublingual tablets, etc.), drug control-release preparations, enteric coated preparations, gastric soluble preparations, etc., that are all prepared in the best form considering the administration route, bioavailability, side effects, etc.

The pharmaceutical composition of the present invention can be orally administered preferably to adult patients aged 15 years or older, regardless of gender. Such patients are patients with clinical conditions that are expected to be improved by fibrinolytic enhancement, and the patients include not only patients whose t-PA activity tends to decrease (fibrinolytic system tends to decrease), but also patients who need to further enhance the fibrinolytic system. The patients include, as described above, not only patients with thrombotic diseases or clinical conditions, but also patients with diseases or clinical conditions related to tissue or organ fibrosis, patients with emphysema diseases or clinical conditions, patients with inflammatory diseases or clinical conditions, and the like. The patients also include patients with intrapulmonary microthrombosis, pulmonary fibrosis, emphysema, and/or respiratory inflammation, including lung inflammation (including cytokine storm).

In particular, patients targeted by the pharmaceutical composition of the present invention include patients with the above symptoms and clinical conditions caused by coronavirus infections such as COVID-19. Preferable are patients who are diagnosed as positive for COVID-19 by the PCR method or antigen test and have evidence of pneumonia by chest CT examination (COVID-19 pneumonia patients), particularly patients with mild or more severe symptoms, preferably mild to moderate symptoms. Examples of such patients include, but are not limited to, nonventilated patients with an SpO₂ value (arterial blood oxygen saturation measured by a pulse oximeter) of less than 95% under room air inhalation at rest (without oxygen inhalation), or patients who require an oxygen concentration of less than 5 L/min (with oxygen inhalation). In particular, such patients have a high risk of aggravation of respiratory inflammation or progression to ARDS; thus, the administration of the pharmaceutical composition of the present invention is highly useful.

The above patients also include those who tend to have the diseases or clinical conditions described above, and whose clinical conditions may further progress.

From the results of the experimental examples provided later, the daily dosage of the pharmaceutical composition of the present invention for an adult patient aged 15 years or older can be within the range of 120 to 300 mg, preferably 120 to 240 mg, in terms of the amount of the present compound, which is an active ingredient. This dosage is a suitable amount that satisfies both effectiveness and safety by orally administering the present compound to the adult patient once daily. Within this range of dosage, while exhibiting both effectiveness and safety, the pharmaceutical composition of the present invention can be continuously administered repeatedly once daily for at least 7 days, preferably at least 14 days, or even for as long as 1 year.

The above dosage is the dosage of a pharmaceutical composition substantially containing the present compound as a single active ingredient. The pharmaceutical composition of the present invention can be used in combination with other pharmaceutical compositions. In that case, the lower limit of the dosage of the present compound can be reduced to about 30 mg/day, and preferably about 60 mg/day. Examples of other pharmaceutical compositions include conventional pharmaceutical compositions (e.g., anticoagulants, thrombolytics, and antiplatelet agents) used for preventing or treating the thrombotic diseases and clinical conditions mentioned above.

In the present specification, the terms "comprise" and "contain" include the meanings of "consisting of" and "consisting essentially of."

### Examples

The present invention is described below with reference to Experimental Examples to help understanding of the structure and effects of the invention. However, the invention is not limited to these Experimental Examples. The following experiments were performed under the conditions of room temperature (25±5°C) and atmospheric pressure, unless otherwise specified. In the following, unless otherwise specified, "%" means "mass%," and "parts" means "parts by mass."

In the following Experimental Examples, sodium 5-chloro-2-({[3-(quinolin-8-yl)phenyl]carbonyl}amino)benzoate (yellowish white powder) was used as the present compound. In the following Experimental Examples, the compound is referred to as "the present compound." The compound was produced according to the disclosure of Example 68 of WO2010/113022.

In the following Experimental Examples, non-clinical studies were performed in accordance with Ordinance No. 21 of the Ministry of Health and Welfare "Ordinance on Standards for Implementation of Non-clinical Studies on Safety of Drugs" (March 26, 1997, partially revised by Ordinance No. 114 of the Ministry of Health, Labour and Welfare, June 13, 2008) and "Guideline on Safety Pharmacology Studies" (PMSB/ELD Notification No. 902, dated June 21, 2001) according to GLP standards. Laboratory animals were handled according to the predetermined guidelines at the contract laboratory ("Act on Welfare and Management of Animals" (Act No. 105 of October 1, 1973, final revision: Act No. 105 of August 30, 2011), "Standards Relating to the Care and Management of Laboratory Animals and Relief of Pain" (April 28, 2006, Notice No. 88 of the Ministry of the Environment), and "Fundamental Guidelines for Proper Conduct of Animal Experiment and Related Activities in Academic Research Institutions" (June 1, 2006, Notice No. 71 of the Ministry of Education, Culture, Sports, Science and Technology)). Clinical studies were performed in compliance with ethical principles based on the Declaration of Helsinki and its revisions, "Ordinance on Good Clinical Practice (GCP)" (Ordinance No. 28 of the Ministry of Health and Welfare, March 27, 1997), which is standards prescribed in Article 14 (3) and Article 80-2 of the "Act on Securing Quality, Efficacy and Safety of Products Including Pharmaceuticals and Medical Devices (PMD Act)," and the requirements for privacy protection of subjects.

### Reference Example

Many patients with severe acute respiratory syndrome associated with COVID-19 develop severe inflammation of the lungs, which often deteriorates into acute respiratory distress syndrome (ARDS), including pulmonary fibrosis and associated pulmonary failure. Before the description of the Experimental Examples, the following summarizes reports so far on the involvement of PAI-1 in ARDS, the usefulness of PAI-1 inhibitors, and the relationship between clinical conditions caused by coronavirus infection and PAI-1.

### (1) Involvement of PAI-1 in ARDS and Usefulness of PAI-1 Inhibitors

Studies using PAI-1-deficient mice, which are gene-deficient animals, and studies using PAI-1-specific inhibitors have revealed that PAI-1 plays an important role in the progression of inflammation, fibrosis, and emphysema.
(a) Reports of Studies Using PAI-1 Gene-deficient Animals
   - Idiopathic pulmonary fibrosis induced by administration of bleomycin does not develop in PAI-1-deficient mice (NPL 7).
   - The lungs of senescence-accelerated klotho mice show an emphysema-like lesion, whereas klotho mice lacking the PAI-1 gene do not show such a lesion (NPL 9).
   - Administration of a tobacco extract does not induce an emphysema-like lesion in PAI-1-deficient mice (NPL 30).
   - L-NAME does not induce an emphysema-like lesion in PAI-1-deficient mice (NPL 31).
(b) Reports of Studies Using PAI-1 Inhibitors
   - A PAI-1 inhibitor inhibited pulmonary fibrosis induced by an adenoviral vector expressing TGFβ (NPL 32).
   - A PAI-1 inhibitor inhibited induction of an emphysema-like lesion by L-NAME (NPL 31).
   - A PAI-1 inhibitor inhibited airway hypersensitivity induced by antigen challenge in mice sensitized to ovalbumin (NPL 33).

The above reports indicate that drugs that inhibit PAI-1 play an important role in the progression of inflammation, fibrosis, and emphysema, and are expected to be useful as, in particular, agents for protecting or treating the lungs of elderly people who have underlying lung damage.

### (2) Relationship between Clinical Conditions Caused by Coronavirus Infection and PAI-1

Many reports suggest a direct relationship between coronaviruses and PAI-1.
- The nucleocapsid protein of coronaviruses interacts with the intracellular signaling molecule Smad3 to increase PAI-1 expression induced by TGFβ (NPL 24). From this report, coronaviruses are considered to directly induce PAI-1 expression, and inhibit apoptosis of infected cells and induce tissue fibrosis by regulating TGFβ signaling.
- The mortality in COVID-19 patients in Wuhan correlates with blood d-dimer (NPL 28). This report showed that the mortality was high in patients with high d-dimer levels (1 ug/mL or more), i.e., patients with a hypercoagulable state. This indicates that the increase in PAI-1 due to coronavirus infection may have been involved in hypercoagulability.
- Moderate to severe lung damage in COVID-19 patients (13 patients) was improved by administration of plasmin (NPL 27). This result indicates that activation of the fibrinolytic system leads to the treatment of ARDS and suggests that PAI-1 inhibition is a therapeutic tool in this mechanism.

The above reports indicate that the PAI-1 inhibitory effect of PAI-1 inhibitors and their fibrinolytic enhancing effect are expected to suppress hypercoagulability and pulmonary fibrosis in COVID-19 patients to thus suppress or improve ARDS effectively.

### Experimental Example 1: Evaluation of Stability of Present Compound

The present compound (yellowish white powder) was prepared in the following shipping packaging form and then subjected to an accelerated stability study (40°C, 75% RH, 6 months).

### Packaging Form

Inner package: A double polyethylene bag (100 × 200 mm) was filled with the present compound and sealed with a zip tie. Outer package: The double polyethylene bag filled with the present compound was placed in a fiber drum, and the fiber drum was sealed.

The present compound was stored in the above packaging form under the conditions of 40°C±2°C and 75% RH±5% RH for 6 months. At points after 1 month, 3 months, and 6 months, appearance observation, an identification test (ultraviolet spectrum, infrared absorption spectrum, flame reaction), water content analysis (water absorption), analysis of the content of the present compound, and a purity test (impurity profile) were performed to evaluate the stability of the present compound.

In all of the items of the accelerated stability study, no change from the state before storage was observed in the present compound at each measurement point, confirming that the properties of the present compound are extremely stable.

### Experimental Example 2: Evaluation of PAI-1 Inhibitory Effect (fibrinolysis Enhancement Effect) (in vitro Study)

A human plasma clot fibrinolysis test was performed to evaluate the PAI-1 inhibitory effect of the present compound.

Specifically, human-derived PAI-1 and the present compound were added to a reaction mixture containing t-PA, which dissolves blood clots caused by addition of thrombin to normal human pooled plasma, and the inhibition of the effect of t-PA by PAI-1 (PAI-1 effect) and the removal of the PAI-1 effect by the present compound (PAI-1 inhibition) (fibrinolysis enhancement) were evaluated.

The results showed that the present compound has a PAI-1 inhibitory effect (fibrinolysis enhancement effect) at 10 µM or more.

### Experimental Example 3: Evaluation of Antithrombotic Effect (in vivo Study)

### (1) Evaluation of Effectiveness in Rat Model of Ferric Chloride-induced Carotid Thrombosis

The effectiveness (antithrombotic effect) of the present compound was evaluated using a rat model of ferric chloride-induced thrombosis

### i. Experimental Method

The evaluation of the antithrombotic effect was performed according to the method described in Test Example 3 of WO2010/113022. Specifically, two hours before the procedure, the present compound suspended in a 0.5% sodium carboxymethylcellulose (CMC-Na) aqueous solution was orally administered to test groups of rats at dosages of 0.3, 1, and 3 mg/kg. To a vehicle group of rats, a 0.5% CMC-Na aqueous solution that did not contain the present compound was orally administered. Under anesthesia, ferric chloride was applied to a carotid artery of each rat to induce thrombus formation by damaging the blood vessel, and the time from thrombus formation to occlusion of the carotid artery (occlusion time) up to 30 minutes was measured with a Doppler blood flowmeter. Occlusion of the blood flow was defined as a state in which the average blood flow indicated zero on the chart.

The bleeding effect was also evaluated by measuring the bleeding time. The bleeding time was measured in the same rats as above by sticking the tail of each rat with an animal lancet 20 minutes after the ferric chloride treatment and then observing the presence or absence of blood adhered to filter paper every 30 seconds. The time until bleeding stopped (bleeding time) was used as an index of bleeding.

### ii. Experimental Results

Fig. 1 shows the evaluation results of the antithrombotic effect (A: occlusion time, B: bleeding time). As shown in Fig. 1, it was confirmed that oral administration of the present compound prolongs the occlusion time in a dose-dependent manner; i.e., the present compound has a thrombus formation inhibitory effect (antithrombotic effect) (see Fig. 1(A)). There was no significant difference in bleeding time between the test groups and the vehicle group (see Fig. 1(B)). From this result, it was considered that the present compound does not have side effects such as a bleeding effect.

### (2) Evaluation of Antithrombotic Effect in Cynomolgus Monkey Model of Saphenous Arterial Thrombus

The thrombus formation inhibitory effect of the present compound was evaluated using a cynomolgus monkey model of saphenous arterial thrombus (PIT model).

### i. Experimental Method

Before the procedure, the present compound suspended in a 0.5% CMC-Na aqueous solution was orally administered to test groups of cynomolgus monkeys at dosages of 0.1, 0.3, 1, and 3 mg/kg. To a vehicle group of cynomolgus monkeys, a 0.5% CMC-Na aqueous solution that did not contain the present compound was orally administered. A light irradiation probe was placed on a saphenous artery of each cynomolgus monkey to cause thrombus formation by applying green light of 540 nm for 20 minutes at the time of intravenously administering rose bengal (20 mg/kg). A decrease in blood flow due to this thrombus formation was measured with a pulse Doppler blood flowmeter, and the thrombus formation inhibitory effect of the present compound was evaluated by measuring the occlusion time on the first day and the cumulative occlusion time. Fig. 2 shows the measurement results of the cumulative occlusion time.

### ii. Experimental Results

Significant prolongation (P<0.05) of the initial occlusion time was observed in the 0.3 mg/kg oral administration group compared with the vehicle group (0 mg/kg) (not shown). In addition, as shown in Fig. 2, a dose-dependent reduction in the cumulative occlusion time was observed; significant reductions (P<0.01) were observed in the 0.1 mg/kg, 0.3 mg/kg, 1.0 mg/kg, and 3.0 mg/kg administration groups compared with the vehicle group (0 mg/kg). This result confirmed that the present compound has an inhibitory effect on thrombus formation (antithrombotic effect). This result also confirmed that the present compound exhibits a dose-dependent fibrinolytic enhancing effect and that the minimum effective dosage of the present compound for oral administration to the monkeys is 0.1 mg/kg.

This minimum effective dosage was as low as 1/3 of the minimum effective dosage (0.3 mg/kg) of a structurally similar compound (2-([biphenyl-3-ylcarbonyl]amino)-5-chlorobenzoic acid) (hereinafter referred to as "the similar compound"), which has PAI-1 inhibitory activity as in the present compound, confirming that the present compound is highly effective.

### Experimental Example 4: Evaluation of Pulmonary Fibrosis-Improving Effect (in vivo Study)

The improvement effect of the present compound on pulmonary fibrosis (remodeling after lung damage) was evaluated using a mouse model of bleomycin-induced pulmonary fibrosis. The mouse model of bleomycin-induced pulmonary fibrosis is an animal model in which a lung epithelial cell disorder, inflammation, and fibrosis are artificially induced by intratracheal administration of bleomycin. Because of similarity of histopathological changes to pulmonary fibrosis in humans, this mouse model is widely used for the evaluation of the drug efficacy.

### i. Experimental Method

To a fibrosis mouse model (fibrosis model group) given a single dose of bleomycin (1.2 mg/mL), the present compound (0.75 mg/kg/day) was orally administered for 21 days (present compound administration group, n = 9), and biochemical analysis of the lungs and measurement of clinical conditions of the lungs were performed on day 22. For comparison, instead of the present compound, the solvent used to dissolve the present compound was administered to a fibrosis model group in the same manner (vehicle group, n = 10), and biochemical analysis and measurement of clinical conditions of the lungs were performed. As a positive control group, a dexamethasone administration group (0.25 mg/kg administration, n = 9) was used. The biochemical analysis was performed using lung hydroxyproline values as an index. The clinical conditions of the lungs were evaluated by performing Masson's trichrome staining and calculating Ashcroft scores according to a report of Ashcroft et al. (Ashcroft T et al., J Clin Pathol, 1988; 41:467). The clinical conditions were confirmed by comparing a normal mouse group (normal group, n = 10) with the vehicle group (n = 10), and the improvement effect of the present compound on pulmonary fibrosis was evaluated by comparing the vehicle group (n = 10) with the present compound administration group (n = 9). Differences between the mean values of the groups were tested by the Bonferroni selected pairs test using Prism 4 (GraphPad Software).

### ii. Experimental Results

Table 1 and Fig. 3(A) and Fig. 3(B) show the results of comparisons of the lung hydroxyproline values and the Ashcroft scores in the groups. The mean values of the groups are expressed as mean±SD, and examination was performed at a 5% significance level.

**Table 1**

| | Normal group (n = 10) | Vehicle group (n = 10) | Dexamethazone administration group (n = 9) | Present compound administration group (n = 9) |
|---|---|---|---|---|
| Lung hydroxyproline value (µg/left lung) | 58.4±5.6 | 74.8117.7 | 71.0±6.7 | 58.7±10.1 |
| Ashcroft score | 0.1±0.0 | 3.1±0.9 | 2.3±0.1 | 2.2±0.3 |

In both results, the vehicle group showed significantly higher values than the normal group.

As shown in Table 1 and Fig. 3(A), there was no significant difference between the lung hydroxyproline value of the vehicle group and the lung hydroxyproline value of the dexamethasone administration group; however, the present compound administration group showed a significantly lower lung hydroxyproline value than the vehicle group, suggesting that the present compound has an antifibrotic effect. Further, as shown in Table 1 and Fig. 3(B), the Ashcroft scores were significantly lower in both the present compound administration group and the dexamethasone administration group than in the vehicle group, and the present compound administration group showed a significant improvement in the Ashcroft score compared with the vehicle group.

These results confirmed that the present compound exhibits an effect of improving pulmonary fibrosis (antifibrotic effect, pulmonary fibrosis therapeutic effect) by oral administration of the present compound.

### Experimental Example 5: Toxicity Study

A repeated dose toxicity study, a mutagenicity study, a chromosomal abnormality study, a micronucleus study, a reproductive and developmental toxicity study, and an embryo-fetal developmental toxicity study were performed for the present compound to evaluate the toxicity of oral administration of the present compound.

### (1) General Toxicity Study (Repeated Dose Toxicity Study) (GLP-compliant)

### (a) Rats

A 4-week repeated oral dose study (GLP-compliant) was performed in rats at dosages of 30, 60, 120, and 250 mg/kg/day. No deaths were found in any of the rats during the administration period. No effect of administration of the present compound was observed on the general condition, body weight, food consumption, ophthalmologic examination, electrocardiography, hematological test, urinalysis, necropsy, or organ weights. Based on these results, the no-observed-adverse-effect level of the present compound in rats was determined to be 250 mg/kg/day for both males and females.

In contrast, the no-observed-adverse-effect level of the structurally similar compound described above in rats was 20 mg/kg/day. This confirmed that the present compound is safer and has a wider safety margin than the structurally similar compound (see Experimental Example 3(2)).

### (b) Monkeys

A GLP-compliant 39-week repeated oral dose study was performed in cynomolgus monkeys at dosages of 10, 30, and 100 mg/kg. No deaths were found in any of the monkeys during the administration period. No effect of administration of the present compound was observed on the general condition, body weight, food consumption, ophthalmologic examination, electrocardiography, hematological test, urinalysis, necropsy, or organ weights.

### (2) Genotoxicity Study

The following studies were performed to confirm that the present compound has no genotoxicity.

### (a) Mutagenicity Study (GLP-compliant)

The gene mutagenicity by the present compound was examined using bacteria.

A study was conducted in the presence of a metabolic activator (hereinafter referred to as a "metabolic activation method") and in the absence of a metabolic activator (hereinafter referred to as a "direct method") by a pre-incubation method, using three strains, i.e., *Salmonella typhimurium* TA100 and TA1535, and *Escherichia coli* WP2uvrA, to detect base pair substitution gene mutations, and two strains, i.e., *Salmonella typhimurium* TA98 and TA1537, to detect frameshift gene mutations. Based on the results of both the metabolic activation method and the direct method, it was determined that the present compound has no mutagenesis effect on any of the five strains.

### (b) Chromosomal Abnormality Study (GLP-compliant)

A study on the clastogenic property of the present compound was performed in the presence of a drug metabolism-activating enzyme (S9 mix) (hereinafter referred to as a "metabolic activation method") and in the absence of a drug metabolism-activating enzyme (S9 mix) (hereinafter referred to as a "direct method") using mammalian cultured cells (Chinese hamster cultured cells (CHL/IU cells)). The results showed that there was no increase in the incidence of structural abnormalities (-gap) or the incidence of ploidy abnormalities (poly) compared with the negative control dimethyl sulfoxide (DMSO) group at any observed dosage, regardless of the presence or absence of metabolic activation. Based on the results, it was determined that the present compound has no clastogenic effect on CHL/IU cells under the conditions of this study.

### (c) Micronucleus Study (GLP-compliant)

The present compound was orally administered to rats to examine its micronucleus-inducing properties and bone marrow cell proliferation suppressive effect on immature bone marrow erythrocytes.

Specifically, the present compound was orally administered to rats twice 24 hours apart, and bone marrow smears were prepared 18 to 24 hours after the last administration. 2000 immature erythrocytes (IEs) were observed per individual, and the percentage of micronucleated immature erythrocytes (micronucleated IEs, MNIEs) contained in the immature erythrocytes [MNIEs (%)] was calculated. In addition, 1000 erythrocytes were observed as the total erythrocyte count per individual, and the percentage of IEs in the total erythrocytes [IEs (%)] was determined. The results showed that the mean value of MNIEs (%) in the present compound administration group was 0.01 to 0.07%, with no significant increase compared with the negative control group (0.08%). The mean value of IEs (%) in the present compound administration group was 55.2 to 62.1%, with no significant decrease compared with the negative control group (56.5%). During the study period, no deaths or changes in the general condition were observed in any of the rats, and there were no variations in body weight compared with the negative control group.

Based on these results, it was determined that under the conditions of this study, the present compound has no micronucleus-inducing effect on immature rat bone marrow erythrocytes and no proliferation suppressive effect on bone marrow cells.

### (3) Reproductive and Developmental Toxicity Study

The effect of oral administration of the present compound on fertility and early embryogenesis up to implantation was examined using rats. Specifically, the present compound was orally administered to male and female rats for 2 weeks before mating, during mating, until the day before necropsy for the males, and until day 7 of gestation for the females, and the effect on fertility and early embryogenesis of the parents was examined. During the study period, the general conditions, body weights, and food consumption of all of the animals were observed and measured, and a fertility test was performed for the males and females. For the females, an estrous cycle test was performed, and a Cesarean section test was performed at day 13 of gestation. In the females, necropsy was performed at the time of Cesarean section, and in the males, necropsy was performed after completion of the Cesarean section of females.

No deaths were found in any of the males and females in the present compound administration group during the observation period, and no abnormalities due to administration of the present compound were observed in the general condition, body weight, food consumption, necropsy, estrous cycle test, fertility test, or Cesarean section test. Based on these results, it was concluded that the no-observed-adverse-effect level of the present compound in the parent animals, including fertility, and the no-observed-adverse-effect level of the present compound with respect to early embryogenesis, under the conditions of this study, are both 1000 mg/kg/day.

### (4) Embryo-fetal Developmental Toxicity Study

The effect of oral administration of the present compound on embryo-fetal development was examined using rats. Specifically, the present compound was orally administered to pregnant rats from implantation to closure of the hard palate (days 7 to 17 of gestation), and the effect on the mothers, and embryos and fetuses was examined. Furthermore, the plasma concentration of the present compound after administration was measured, and its change was examined. During the study period, the general conditions of all of the animals were observed, and their body weights were measured; in the toxicity group, food consumption was measured; a Cesarean section test was performed on day 20 of gestation. Thereafter, fetal visceral and skeletal specimens were prepared, and a fetal examination was performed.

Regarding the effect on the mothers, no deaths were found during the observation period, and no abnormalities due to administration of the present compound were observed in the general condition, body weight, food consumption, or necropsy.

Regarding the effect on embryo-fetal development, no abnormalities due to administration of the present compound were observed in the number of corpora lutea, number of implanted embryos, pre-implantation mortality, post-implantation mortality (early embryonic resorption rate, late embryonic resorption rate, and dead fetus rate), number of live fetuses, sex ratio, live fetal body weight, or placental findings. No abnormalities were observed in an external examination of the fetuses, and no abnormalities due to administration of the present compound were observed in visceral or skeletal examination.

Based on the above results, it was considered that oral administration of the present compound has no effect on maintenance of pregnancy, no embryolethal effect, no fetal growth suppressive effect, and no teratogenic effect. Thus, it was concluded that the no-observed-adverse-effect levels of the present compound for the mothers, and embryos and fetuses under the conditions of this study are both 1000 mg/kg/day.

### Experimental Example 6: Safety Pharmacology Study (GLP-compliant)

A safety pharmacology study of the present compound was performed to evaluate its effect on hERG current and its effect on the central nervous system, the cardiovascular system, and the respiratory system.

### (1) Effect on hERG Current

The effect of the present compound on hERG current was examined by a whole-cell patch clamp method using HEK293 cells transfected with the human ether-a-go-go-related gene (hERG). This study was performed as part of a safety pharmacology core battery study to predict the risk of a drug-induced proarrhythmic effect, in particular, prolongation of QT interval on an electrocardiogram, in an *in vitro* study. The concentration of the present compound was set to 0.1, 1, and 10 µM, and the tail peak current suppression rate (%) 10 minutes after application relative to that before application was used as an index of the effect on hERG current. As a vehicle control, 0.1 v/v% DMSO was used, and as a positive control, 0.1 µmol/L of E-4031 was used.

The results showed that the suppression rates in the 0.1, 1, and 10 µM groups of the present compound were not different from that of the vehicle control group, confirming that the present compound has no suppressive effect on hERG current in hERG-expressing HEK293 cells up to 10 µM; i.e., the present compound has no effect on hERG current.

### (2) Effect on Central Nervous System

The effect of the present compound on the central nervous system was examined by the functional observational battery (FOB) in rats, using male Crl:CD(SD) rats. Specifically, the present compound was orally administered to the rats in a single dose, and the rats were observed before administration and 0.5, 1, 2, 4, 8, and 24 hours after administration. To a control group (six rats), a 0.5 w/v% carmellose sodium aqueous solution (vehicle) was administered instead of the present compound in the same manner. The results showed that no changes due to administration of the present compound were observed in any of FOB observation items. Thus, it was considered that the present compound has no effect on the central nervous system up to a dosage of 300 mg/kg in single oral dose administration using rats.

### (3) Effect on Cardiovascular System and Respiratory System

The effect of the present compound on the cardiovascular system and the respiratory system was examined by a telemetry method and blood gas measurement using conscious cynomolgus monkeys. Systemic exposure of the test substance was also evaluated. Specifically, a control substance (a 0.5 w/v% carmellose sodium aqueous solution) and the present compound were orally administered at single ascending dosages at intervals of 7 days, and the heart rate, blood pressure (systolic blood pressure, diastolic blood pressure, and mean blood pressure), electrocardiogram (PR interval, QRS duration, QT interval, and QTc interval), respiratory rate, and body temperature were measured before administration and 1, 2, 4, 6, and 24 hours after administration on each administration day with the monkeys unanesthetized and unrestrained. Furthermore, before administration and 2, 6, and 24 hours after administration on each administration day, arterial blood was collected, and blood gases (oxygen partial pressure, carbon dioxide partial pressure, pH, and oxygen saturation) were measured. Systemic exposure was also evaluated by measuring the concentration of the test substance in plasma.

The results showed that no variations due to administration of the present compound were observed in the heart rate, blood pressure (systolic, diastolic, and mean), electrocardiogram parameters (PR interval, QRS duration, QT interval, and QTc interval), body temperature, respiratory rate, or blood gas parameters (oxygen partial pressure, carbon dioxide partial pressure, pH, and oxygen saturation). In addition, no abnormalities in the general condition were observed. Thus, it was considered that in single oral dose administration using cynomolgus monkeys, the present compound has no effect on the cardiovascular system or the respiratory system up to a dosage of 300 mg/kg.

### Experimental Example 7: Clinical Study (Phase I Study/Single Dose)

### (1) Placebo-controlled Single Oral Dose Study in Healthy Adult Males

A placebo-controlled single oral dose study was performed to examine the safety and pharmacokinetics upon single oral dose administration of the present compound in healthy Japanese adult males (subjects). The details are shown in Table 2.

**Table 2**

| Summary of phase I study/placebo-controlled single oral dose study | | |
|---|---|---|
| Study method | Single oral dose | |
| | The present compound (30, 60, 120, or 240 mg) or a placebo is orally administered at a single dose to 8 healthy adult male subjects (6 subjects in the present compound administration group and 2 subjects in the placebo group) per step with the subjects fasting. To examine the food effect, the present compound (60 mg) is orally administered at a single dose to each of 6 healthy adult male subjects (5 subjects in the present compound administration group and 1 subject in the placebo group) after a meal. | |
| | The subjects are randomly assigned, and the study is performed as a double-blind study. | |
| | The dosage is increased after confirming that there are no safety issues in each step. | |
| Number of subjects | 32 subjects (a total of five steps) | |
| | Step 1 (30 mg): 8 subjects | |
| | Step 2 (60 mg): 8 subjects | |
| | Step 3 (120 mg): 8 subjects | |
| | Step 4 (food effect (60 mg)): 6 subjects | |
| | Six of the subjects who participate in step 2 participate in step 4. | |
| | Step 5 (240 mg): 8 subjects | |
| Key inclusion criteria | Subjects who meet all of the following criteria in confirmation or measurement and examination at the time of the screening test and at the time of admission are included in this clinical trial. | |
| | 1. Sex: Japanese male | |
| | 2. Age (at the time of obtaining consent): aged 20 or older and younger than 45 | |
| | 3. Body weight: 50.0 kg or more and less than 85.0 kg | |
| | 4. BMI: 18.5 or more and less than 25.0 | |
| | 5. Written consent is obtained. | |
| Key exclusion criteria | Subjects who meet any of the following criteria in confirmation or measurement and examination at the time of the screening test and at the time of admission are excluded from this clinical trial. | |
| | 1. Subjects with a history of any of the following: | |
| | | (1) liver disease (e.g., viral hepatitis or drug-induced hepatic injury) |
| | | (2) heart disease (e.g., congestive heart failure, angina pectoris, or arrhythmia requiring treatment) |
| | | (3) respiratory disease (e.g., severe bronchial asthma or chronic bronchitis) |
| | | (4) digestive system disease (e.g., severe peptic ulcer, reflux esophagitis, or diseases requiring various types of resection, excluding appendicitis and hemia) |
| | | (5) kidney disease (e.g., acute renal failure, glomerulonephritis, or interstitial nephritis) |
| | | (6) cerebrovascular disorder (e.g., cerebral infarction) |
| | | (7) malignancy |
| | 2. Subjects with a history of drug allergy or food allergy and idiosyncrasy | |
| | 3. Subjects who have received treatment including administration of a drug (including a supplement) within 14 days before the screening test | |
| | 4. Subjects to whom a drug has been administered in another clinical trial or a postmarketing clinical study within 120 days before the screening test | |
| | 5. Subjects from which 1200 mL or more of whole blood has been collected within 1 year before the screening test, from which 400 mL or more of whole blood has been collected within 84 days before the screening test, from which 200 mL or more of whole blood has been collected within 28 days before the screening test, or who have made a blood component donation within 14 days before the screening test | |
| | 6. Subjects who have smoked or used a nicotine-containing preparation | |
| | 7. Subjects with abnormal results of an immunological test (HBs antigen, HCV antibody, test for syphilis (STS, TP antibody), HIV antigen/antibody) | |
| | 8. Subjects with drug dependence (including those with abnormal urine drug test results) or alcohol dependence (including those with a history of alcohol dependence) | |
| | 9. Subjects who are bleeding, who have a predisposition or tendency to bleeding, who have a history of bleeding, or who have a family history of a bleeding disorder | |
| | 10. Subjects who are supposed to receive instructions and orders from the principal investigator or are employed by the clinical trial medical institution | |
| | 11. Subjects deemed inappropriate for participation in this clinical trial by the principal investigator (or subinvestigator) | |
| Administration method | 30 mg, 60 mg, 120 mg, or 240 mg of the present compound or a placebo was orally administered at a single dose with the subjects fasting or after a meal (food effect (60 mg) only). | |

### i. Study Method

The oral dosages were 30 mg (step 1), 60 mg (step 2), 120 mg (step 3), and 240 mg (step 5) per day, and the food effect was separately examined in administration at a dosage of 60 mg (step 4) (a total of five steps). In steps 1 to 3 and 5, the number of subjects per step was 8 (6 subjects in the present compound administration group and 2 subjects in the placebo group), of which 1 subject of the present compound administration group and 1 subject of the placebo group were used as the preceding group, and 5 subjects of the present compound administration group and 1 subject of the placebo group were used as the following group. The study proceeds to the following group after confirming safety in the preceding group. The transition from one step to the next was made after confirming safety. In step 4, which examines the food effect, the present compound was orally administered to all 6 subjects. In the steps other than step 4, the present compound or the placebo was orally administered with the subjects fasting (before breakfast), and in step 4, oral administration was performed after a meal (after breakfast).

After administration of the present compound and the placebo, the following items were evaluated in each subject.
Safety: vital signs (axillary temperature, blood pressure while seated, and pulse rate while seated), 12-lead electrocardiogram, laboratory tests (hematological test, blood biochemical test, blood coagulation test, urinalysis, and fecal test), and adverse events
Pharmacokinetics: concentration of unchanged drug in plasma and metabolite search, and concentration of unchanged drug in urine and metabolite search (only in administration at a dosage of 120 mg) .

### ii. Study Results

All of the subjects completed the study, and no subjects were withdrawn from the study. No adverse events causally related to administration of the present compound were observed throughout the study. The results of the pharmacokinetics study confirmed that the AUC showed linearity up to a dosage of 240 mg of the present compound. The single oral dose study in the healthy subjects confirmed that the present compound does not pose a safety issue at a daily oral dosage of 30 mg to 240 mg.

### Experimental Example 8: Clinical Study (Phase I Study/Repeated Dose)

### (1) Placebo-controlled Repeated Oral Dose Study in Healthy Adult Males

A placebo-controlled repeated oral dose study was performed to examine the safety, pharmacokinetics, and pharmacodynamics of the present compound during repeated oral dose administration in healthy Japanese adult males (subjects). The details are shown in Table 3.

**Table 3**

| Summary of phase I study/placebo-controlled repeated oral dose study | |
|---|---|
| Study method | Repeated oral dose |
| | The present compound (60 or 120 mg) or a placebo was orally administered to 9 healthy adult male subjects (6 subjects in the present compound group and 3 subjects in the placebo group) per step once daily after breakfast for 7 days. |
| | The investigational drug is randomly assigned, and the study is performed as a double-blind study. |
| | The dosage is increased to 120 mg after confirming that there are no safety issues in administration at a dosage of 60 mg. |
| Number of target subjects | Total: 18 subjects (a total of 2 steps) |
| | - Step 1: 9 subjects (60 mg) |
| | - Step 2: 9 subjects (120 mg) |
| Key inclusion criteria | Same as in Table 2 |
| Key exclusion criteria | -Same as in Table 2. |
| | -Subjects who received this drug in other clinical studies. |
| Dosage, administration method, and administration period | Dosage: 60 mg, 120 mg |
| | Administration method: oral administration after meals |
| | Administration period: once daily for 7 days |
| Combination treatment | In principle, combination treatment (use of drugs other than the investigational drug and therapy) except for treatment for adverse events is prohibited during the clinical trial period. |

### i. Study Method

The oral dosages were 60 mg (step 1) and 120 mg (step 2) per day, and the number of subjects per step was 9 (6 subjects in the present compound group and 3 subjects in the placebo group). The transition from one step to the next was made after confirming safety. The present compound or the placebo was orally administered once daily after breakfast for 7 days.

After administration of the present compound and the placebo, the following items were evaluated in each subject. Safety: vital signs (axillary temperature, blood pressure while seated, and pulse rate while seated), 12-lead electrocardiogram, laboratory tests (hematological test, blood biochemical test, blood coagulation test, urinalysis, and fecal test), and adverse events

### Pharmacokinetics: plasma concentration of the unchanged drug and metabolite concentration

### Pharmacological activities: PAI-1 activity and t-PA activity

The pharmacological activities (PAI-1 activity and t-PA activity) were evaluated using blood collected from the subjects before and after administration of the present compound or the placebo as test samples. Blood was collected before administration and 2, 4, and 8 hours after administration on the first day of the experiment (day 1) and day 7 after the start of the experiment, and on the other days, blood was collected once a day before administration (only before administration on days 2 to 6, day 8, and day 11 after the start of administration).

### (1) Measurement of PAI-1 activity

After collecting blood samples from the subjects, the samples were treated with 3.2% citric acid salt and centrifuged at 4°C, 1,800 g for 10 minutes to obtain plasma. The collected plasma was used for measurement of PAI-1 activity and stored frozen at -80°C or less until measurement.

The PAI-1 activity was measured using a human PAI-1 activity assay kit (produced by Molecular Innovations Inc.). In this system, a plasma sample collected from a subject is placed on a plate coated with urokinase plasminogen activator (uPA), PAI-1 in the sample is reacted with uPA, and then active PAI-1 bound to uPA is measured as PAI-1 activity using an antigenantibody reaction with an anti-PAI-1 antibody by measuring the absorbance at a wavelength of 450 nm with a plate reader.

### (2) Measurement of t-PA activity

After collecting blood samples from the subjects, the samples were treated with 0.5 M sodium citrate (pH of 4.3) and centrifuged at 4°C, 2,500 g for 15 minutes to obtain plasma. The collected plasma was used for measurement of t-PA activity and stored frozen at -80°C until measurement.

The t-PA activity was measured using a ZYMUPHEN tPA Activity kit (produced by HYPHEN BioMed). In this system, a plasma sample collected from a subject is reacted with human plasminogen to form plasmin by the action of tPA, the plasmin is reacted with a substrate specific to plasmin, and pNA, which is a degradation product produced, is measured by the absorbance at a wavelength of 405 nm with a plate reader.

### ii. Study Results

### (a) Safety Evaluation and Pharmacokinetics Evaluation

All of the subjects completed the study, and no subjects were withdrawn from the study. Throughout the study, oral paresthesia (1 case) was observed as an adverse event causally related to administration of the present compound, but disappeared without treatment. The results of pharmacokinetics study showed that AUC₀₋₂₄ₕ was dose-dependent and that Tₘₐₓ, t_{1/2 0}-₂₄ₕ, and C_{max'} were not affected by repeated administration. AUC increased on day 7 compared with that on day 1. Cₘᵢₙ (trough value) reached a steady state on day 4 in administration at a dosage of 60 mg/day. In administration at a dosage of 120 mg/day, a steady state was estimated to be reached on day 7 based on PK simulation.

This repeated oral dose study in the healthy subjects confirmed that there are no safety issues even when the present compound is orally administered at a daily dosage of 60 mg to 120 mg for 1 week.

### (b) Pharmacological Activity Evaluation (PAI-1 Activity and t-PA Activity)

The results of PAI-1 activity and t-PA activity measured in blood in the repeated oral dose study are shown in Fig. 4 (upper graph: PAI-1 activity (relative value); lower graph: t-PA activity (relative value)). Focusing on the results on days 1 and 7, in which PAI-1 activity and t-PA activity were measured before and after oral administration of the present compound, neither PAI-1 activity nor t-PA activity was different in administration of the present compound at 60 mg/day from that of administration of the placebo, as shown in Fig. 4. However, it was confirmed that administration of the present compound at a dosage of 120 mg/day decreased PAI-1 activity and increased t-PA activity. It was also confirmed that the changes (decrease in PAI-1 activity and increase in t-PA activity) were both maximal 8 hours after administration of the present compound, and then these activities gradually returned to the same levels as those in the placebo administration group on the following day. The activity values before administration remained almost unchanged during the period of day 2 to day 6, and the same changes were observed on both day 1 and day 7, indicating that there was no accumulation of the present compound in the body and no development of resistance.

These results confirmed that the present compound significantly inhibits PAI-1 and enhances t-PA (fibrinolytic enhancement) when orally administered to humans at a dosage of at least 120 mg per day (effectiveness) and that the present compound is safe even when repeatedly administered orally at a dosage of at least 120 mg to 240 mg/day (safety).

### Experimental Example 9: Clinical Study (Phase II Study:

### Evaluation of Safety of Present Compound When Combined with Tyrosine Kinase Inhibitor in Chronic-Phase Chronic Myeloid Leukemia (CML) and Evaluation of Effectiveness against CML)

The present compound and a tyrosine kinase inhibitor (hereinafter referred to as "TKI") were used in combination and repeatedly administered orally to patients with chronic-phase chronic myeloid leukemia (CML patients) to evaluate the safety of the present compound and the effectiveness of the present compound against CML.

The details are shown in Table 4. Specifically, to 21 chronic-phase CML patients treated orally with a TKI for two years or more, 120 mg of the present compound was orally administered once daily after breakfast for 4 weeks in combination with a TKI (imatinib (100 to 800 mg/day), nilotinib (150 to 800 mg/day), or bosutinib (200 to 500 mg/day)), followed by an observation period of 8 weeks. Oral administration of the TKI, which is a drug used in combination, was continued for a total of 12 weeks, including the 8-week observation period. The safety analysis set (SAS) and the full analysis set (FAS) each consisted of the 21 patients, and the per protocol set (PPS) consisted of 18 patients.

**Table 4**

| Phase II study | |
|---|---|
| **Clinical trial design and clinical trial method** | **(1) Clinical trial design** |
| | Open-label, uncontrolled, multicenter study |
| | **(2) Treatment period** |
| | **Administration period: 4 weeks, observation period after administration: 8 weeks, total: 12 weeks** |
| **Number of subjects** | **21** enrolled subjects |
| **Inclusion criteria** | **(1)** Patients aged 18 or older |
| | **(2)** Patients whose written consent for participation in this clinical trial has been obtained from the patients (or a legal representative) |
| | **(3) Patients diagnosed with chronic-phase chronic myeloid leukemia and positive for major BCR-ABL1 expression** |
| | **(4) Patients who each have been treated with a TKI for 2 years or more** |
| | **(5) Patients in which the TKI has not been changed for 12 weeks before enrollment** |
| | **(6) Patients in which the dosage of the TKI has not been changed for 12 weeks before enrollment** |
| | **(7) Patients with a BCR-ABL^{IS} level of 0.1 % or less** |
| | **(8) Patients with a BCR-ABL^{IS} level of more than 0.0032%** |
| | **(9) Patients with an ECOG grade of 0 to 2** |
| | **(10) Patients in which AST and ALT levels are 2.5 times or less the** upper limit of **the normal standard of each medical facility** |
| | **(11) Patients in which the total bilirubin level is 2.5 times or less the** upper limit of **the normal standard of each medical facility** |
| | **(12) Patients who have agreed to use an appropriate method of** contraception during the clinical trial **period.** |
| **Exclusion criteria** | **(1)** Pregnant or lactating women |
| | **(2) Patients who have participated in another clinical study within 30 days before enrollment** |
| | **(3) Patients with a history of hematopoietic stem cell transplantation (autologous or allogeneic cells)** |
| | **(4) Patients with any of the following cardiovascular diseases:** |
| | **- NYHA class II to IV congestive heart failure** |
| | **-A history of myocardial infarction within 6 months before enrollment** |
| | **-** Symptomatic arrhythmia requiring treatment |
| | **- QTc of 480 msec or more** |
| | **(5) Patients with T3151 mutation in the BCR-ABL kinase domain** |
| | **(6)** Patients who have taken **dasatinib within 12 weeks before enrollment** |
| | **(7)** Patients who have newly taken **pioglitazone (Actos or the like) within 12 weeks before enrollment** |
| | **(8) Patients who have been diagnosed as being in an accelerated phase or blast phase in the past** |
| | **(9) Patients** with an active malignancy |
| | **(10) HIV-positive patients** |
| | **(11) Patients with bleeding tendency** |
| | **(12) Other patients deemed inappropriate for inclusion in this clinical trial by the principal investigator (or subinvestigator)** |
| **Investigational drug, dosage, and administration method** | **120 mg of the present compound was orally administered once daily after breakfast for 4 weeks. Administration of the TKI, which is a drug used in combination, was continued for 12 weeks. In principle, the TKI administered or its dosage and administration were not changed during the observation period.** |
| **Combination treatment** | **Drugs prohibited from being used in combination** |
| | **The following drugs are prohibited from being used in combination during the clinical trial period: dasatinib, warfarin potassium, dabigatran etexilate methanesulfonate, rivaroxaban, apixaban, edoxabantosilate hydrate, heparin, and tissue plasminogen *activator* (t-PA)** |
| | **Restricted drugs for combination use** |
| | **The following drug can be used in combination during the clinical trial period if it has been used for diabetes before the start of the clinical trial: pioglitazone** |

No adverse events causally related to the investigational drug occurred in any of the 21 patients in the safety analysis set (SAS). This confirmed that the present compound is safe even when repeatedly administered orally not only to healthy subjects, but also to cancer patients undergoing anticancer treatment (TKI treatment in the above example), at a dosage of at least 120 mg/day for 4 weeks. The results confirmed that the present compound can be used in combination with other therapeutic drugs.

It was confirmed that the cumulative achievement rate of patients who reached a deep molecular response (DMR), defined as MR 4.5 (BCR-ABL^{IS}≤0.0032%), which is an index of anticancer effectiveness for CML patients (DMR achievement rate) (see NCCN Clinical Practice Guideline in Oncology), increased when the present compound was used in combination with the TKI.

### Experimental Example 10: Clinical Study (Phase II Long-term Administration Study: Evaluation of Pharmacokinetics and Safety of Present Compound When Used in Combination of TKI for Long Period of Time in Chronic-phase CML, and Efficacy against CML

In consideration of the results of Experimental Example 9, the present compound and a TKI (imatinib (100 to 800 mg/day), nilotinib (150 to 800 mg/day), dasatinib (20 to 100 mg/day), or bosutinib (200 to 500 mg/day)) were used in combination (both administered orally) in chronic-phase CML patients for 48 weeks to evaluate the pharmacokinetics and safety of the present compound in this study. Further, the efficacy of the combined use of the present compound and the TKI against CML was evaluated.

The summary of the study is almost the same as in Experimental Example 9, except for the following points.

The present compound was orally administered after breakfast to chronic-phase CML patients (33 patients) aged 20 or older and younger than 80 who had been treated orally with the TKI for 1 year or more. The dosage of the present compound was started at 150 mg/day. When the BCR-ABL^{IS} level did not reach a DMR (0.0032% or less) 4 weeks after administration, the dosage could be increased, after the predetermined examination, to 180 mg/day from week 8. No patients dropped out or were withdrawn from the study, and all of the patients were included in analysis (SAS analysis, FAS analysis, and PPS analysis). The dosage was increased to 180 mg/day in 18 of the 27 patients who did not reach a DMR at week 4.

In pharmacokinetics, the blood concentration of the unchanged present compound was measured. The results showed that the concentration of the unchanged present compound remained within a constant range from week 4 to week 48 in each patient and that no accumulation in the body was observed.

In the safety evaluation, eight adverse events possibly causally related to the present compound were observed in 6 patients (18.2%) (lymphopenia, palpebral edema, conjunctival hemorrhage, stomatitis, mouth hypersensitivity, liver function abnormality, and headache), but none of the adverse events required treatment.

These results confirmed that the present compound is safe even when repeatedly administered orally not only to healthy subjects, but also to cancer patients undergoing anticancer treatment (TKI treatment in the above example) at a dosage of at least 150 to 180 mg/day for 48 hours.

### Efficacy against CML

The efficacy against CML was evaluated from the following two points:
(1) cumulative achievement rate of CML patients who reached a DMR (BCR-ABL^{IS}: 0.0032% or less)
(2) change in BCR-ABL^{IS}

Within 28 days before the start of administration of the present compound, and 4, 8, 12, 24, 36, and 48 weeks after the start of administration of the present compound or at discontinuation, the investigator collected peripheral blood and conducted a test using a BCR-ABL^{IS} quantitative PCR kit (produced by Sysmex Corporation) to obtain results (IS%, ABL copy number, and BCR-ABL copy number). From these results, the above points (1) and (2) were evaluated.

Table 5 shows the cumulative achievement rate of CML patients who reached a DMR.

**Table 5**

| **Time** | **Number of patients measured** | **Cumulative number of patients who achieved a DMR** | **Cumulative rate of patients who achieved a DMR** | **95% confidence interval** |
|---|---|---|---|---|
| **Baseline** | **33** | **0** | **0** | **0.0 to 10.6** |
| **4 weeks after the start of administration** | **33** | **5** | **15.2** | **5.1 to 31.9** |
| **8 weeks after the start of administration** | **33** | **9** | **27.3** | **13.3 to 45.5** |
| **12 weeks after the start of administration** | **33** | **9** | **27.3** | **13.3 to 45.5** |
| **24 weeks after the start of administration** | **33** | **10** | **30.3** | **15.6 to 48.7** |
| **36 weeks after the start of administration** | **33** | **11** | **33.3** | **18.0 to 51.8** |
| **48 weeks after the start of administration** | **33** | **11** | **33.3** | **18.0 to 51.8** |

Of the 33 patients, 11 patients achieved a DMR, and the cumulative DMR achievement rate was 33.3% from week 36 onward. This achievement rate is higher than that of a historical control (8%) and is a result that meets the target value (endpoint) of this study (33%).

The details of the dosages of the TKI and the present compound for the 11 patients who achieved a DMR are shown below.

**Table 6**

| | Kind of TKI | Dosage of TKI (mg/day) | Dosage of present compound (mg/day) |
|---|---|---|---|
| 1 | Imatinib | 200 | 150 mg/day continuous |
| 2 | Nilotinib | 150 | 150 mg/day to 180 mg/day increase |
| 3 | Nilotinib | 300 | 150 mg/day to 180 mg/day increase |
| 4 | Nilotinib | 400 | 150 mg/day continuous |
| 5 | Nilotinib | 400 | 150 mg/day to 180 mg/day increase |
| 6 | Nilotinib | 600 | 150 mg/day continuous |
| 7 | Nilotinib | 600 | 150 mg/day continuous |
| 8 | Nilotinib | 600 | 150 mg/day to 180 mg/day increase |
| 9 | Dasatinib | 100 | 150 mg/day continuous |
| 10 | Nilotinib | 100 | 150 mg/day to 180 mg/day increase |
| 11 | Bosutinib | 500 | 150 mg/day continuous |

As shown above, in this study, a significant effect was observed in repeated administration of the present compound (150 to 180 mg/day) and the TKI (imatinib (200 mg/day), nilotinib (150 to 600 mg/day), dasatinib (100 mg/day), or bosutinib (500 mg/day)) for 36 weeks or more.

Regarding the change in BCR-ABL^{IS}, the BCR-ABL^{IS} levels (mean ± standard deviation, Wilcoxon test) at the baseline and weeks 4, 8, 12, 24, 36, and 48 in the 33 patients were 0.0181±0.01764%, 0.0134±0.01301% (P = 0.1222), 0.0129±0.01569% (P = 0.0929), 0.0133±0.02012% (P = 0.0228), 0.0149±0.02596% (P = 0.0427), 0.0152±0.03427% (P = 0.0304), and 0.0210±0.06718% (P = 0.0124). A significant decrease was observed from week 12 onward. According to the administration patterns, a significant decrease was observed at weeks 12 and 24 in the 15 patients who were continuously given the present compound at a dosage of 150 mg/day, and a significant decrease was observed at week 48 in the 18 patients who were given the present compound at dosages of 150 mg/day to 180 mg/day (the dosage being increased).

These results confirmed that the therapeutic effect of the present compound on CML is attained by administering the present compound to CML patients at a dosage of at least 150 to 180 mg/day daily for 36 weeks or more continuously in combination with a TKI. Thus, the present compound, when used at this dosage in combination with a TKI, is considered to exhibit an effect of aiding or reinforcing the therapeutic effect of the TKI on CML while ensuring safety for CML patients. A combination therapy of the present compound with a TKI can be effective as a therapy for curing CML (maintaining complete remission).

### Experimental Example 11: Effectiveness Study for COVID-19 Pneumonia (Exploratory Early Phase II Study)

The effect of the present compound to prevent the aggravation of COVID-19 pneumonia and its safety were explored.

### (1) Summary of Study

**Table 7**

| Clinical trial design | Multicenter, single-arm, open-label, uncontrolled study |
|---|---|
| | Administration period: 14 days |
| Target patients | 26 patients with COVID-19 pneumonia (mild to moderate) |
| Inclusion criteria | 1. Inpatients over 20 years of age with written consent |
| | 2. COVID-19 positive by PCR method or antigen test |
| | 3. Chest CT scan with findings consistent with novel coronavirus pneumonia |
| | 4. Patients with SpO₂<95% under room air inhalation at rest (without oxygen inhalation) or patients who require an oxygen concentration of less than 5 L/min (with oxygen inhalation) |
| | 5. Non-ventilated patients |
| | 6. AST and ALT levels are 2.5 times or less the upper limit of the normal standard of each medical facility |
| | 7. The total bilirubin level is 2.5 times or less the upper limit of the normal standard of each medical facility |
| | 8. Creatinine clearance (calculated by the Cockcroft-Gault formula) is 30 mL/min or more. |
| Exclusion criteria (at the time of enrollment) | 1. Patients on home oxygen therapy |
| | 2. Kidney disease patients on dialysis treatment |
| | 3. Patients with a history or complication of malignancy (excluding those with no recurrence or no new onset of malignancy for at least 5 years after treatment) |
| | 4. Patients with liver cirrhosis (Child-Pugh score class B and C) |
| | 5. Pregnant or lactating patients |
| | 6. Patients who have participated in or are currently participating in another clinical trial or clinical study with intervention within 30 days before enrollment in this study |
| | 7. Patients with bleeding tendency |
| | 8. Patients receiving anticoagulants or other drugs prohibited from being used in combination that are difficult to discontinue |
| | Patients deemed inappropriate for inclusion by the physician for other reasons |
| Dosage and administration | The present compound (tablet) is orally administered once daily after breakfast for 14 days. |
| | Administration is started from 120 mg/day, and on the 7th day, it is confirmed whether the |
| method | dosage can be increased. If the physician determines that the dosage can be increased, the dosage is increased to 180 mg/day on the following day. |
| | The final dosage was 120 mg/day for 2 of the 26 target patients and 180 mg/day for 24. |
| Drugs prohibited from being used in combination | The following drugs are prohibited from being used in combination during the study period. |
| | - Warfarin potassium, - dabigatran etexilate methanesulfonate, - rivaroxaban, apixaban, argatroban hydrate, edoxabantosilate hydrate, heparin, low-molecular-weight heparin, t-PA, nafamostat, camostat, danaparoid sodium, fondaparinux, and drugs under development for the treatment of COVID-19 pneumonia or ARDS (excluding antivirals, such as anti-IL-6 receptor antibodies and JAK inhibitors). |

### (2) Endpoints

### (i) Primary Efficacy Endpoint

Occurrence of transition to oxygen supply treatment under mechanical ventilation after the oral administration of the present compound (occurrence of deterioration of oxygenation)

### (ii) Secondary Efficacy Endpoint

(a) Survival for 28 days from the start of administration of the present compound
(b) Duration of hospitalization after the start of administration of the present compound
(c) Number of days required for oxygen administration after the start of administration of the present compound
(d) Changes in the proportion of lesions in the lung field on chest CT images before and after the administration of the present compound

### (iii) Safety Endpoint

Occurrence of adverse events after the start of administration of the present compound until the end of observation

### (3) Evaluation Results

### (i) Primary Efficacy Endpoint

There were no cases of deterioration of oxygenation requiring mechanical ventilation after the oral administration of the present compound.

### (ii) Secondary Efficacy Endpoint

(a) Survival for 28 days from the start of administration of the present compound: all cases survived.
(b) Duration of hospitalization after the start of administration of the present compound

**Table 8**

| **Duration (days)** | **Number of cases*** |
|---|---|
| **8 to 12 days** | **5** |
| **13 to 17 days** | **12** |
| **18 to 22 days** | **4** |

| | |
|---|---|
| ***: excluding undescribed cases and discontinued cases** | |

(c) Number of days required for oxygen administration after the start of administration of the present compound (number of cases requiring oxygen administration)

**Table 9**

| **Oxygen dosage (Uday)** | **Days** | | | | | | | | | | | | | |
|---|---|---|---|---|---|---|---|---|---|---|---|---|---|---|
| | **1** | **2** | **3** | **4** | **5** | **6** | **7** | **8** | **9** | **10** | **11** | **12** | **13** | **14** |
| **0** | **3** | **3** | **4** | **7** | **7** | **10** | **10** | **12** | **13** | **17** | **18** | **20** | **21** | **22** |
| **2 or less** | **12** | **12** | **13** | **11** | **12** | **9** | **9** | **10** | **9** | **6** | **5** | **3** | **2** | **1** |
| **More than 2 less than 5** | **5** | **6** | **6** | **5** | **4** | **4** | **4** | **1** | **1** | **0** | **0** | **0** | **0** | **0** |
| **5 or more** | **3** | **2** | **0** | **0** | **0** | **0** | **0** | **0** | **0** | **0** | **0** | **0** | **0** | **0** |

The number of days required for oxygen administration after the start of administration of the present compound gradually decreased.
(d) Changes in the proportion of lesions in the lung field on chest CT images before and after the administration of the present compound

The results are shown in Fig. 5. Fig. 5 shows the results of 18 cases, excluding unenrolled cases and discontinued cases. In 13 out of the 18 cases, it was confirmed that the proportion of lesions in the lung field was reduced by the administration of the present compound for 14 days, and it was confirmed that pneumonia was improved or its aggravation was suppressed.

### (iii) Safety Endpoint

From the start of administration of the present compound to the end of observation, 33 adverse events occurred in 17 patients. However, these were constipation, nasal hemorrhage, bloody sputum, headache, and eczema, and there were no adverse events that were related to the present compound and that required treatment. In the study, combined use with heparin was prohibited, whereas combined use with Remdesivir, Avigan, or steroid was allowed. As a result, no adverse events requiring treatment were observed in patients concomitantly treated with Remdesivir (8 cases), Avigan (5 cases), inhaled steroid (22 cases), or antiplatelet agent (5 cases).

As shown in the above study results, there were no adverse events requiring treatment that could be causally related to the present compound. It was thus confirmed that the present compound is safe and effective in preventing the aggravation of COVID-19 pneumonia (deterioration of oxygenation) in COVID-19 pneumonia patients.

Based on Experimental Examples 9 and 10 described above, this patent application also includes the following inventions:
(A) A TKI combination drug to be used in combination with a tyrosine kinase inhibitor (TKI), comprising a compound represented by Formula (1) (hereinafter referred to as "present compound (1)") or a pharmaceutically acceptable salt thereof as an active ingredient: 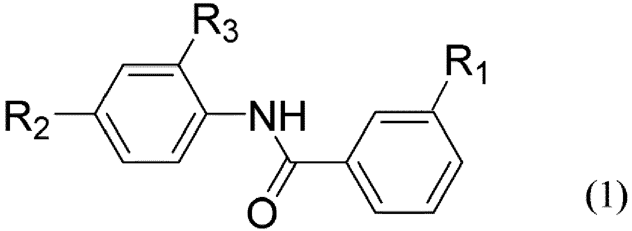 wherein R₁ is quinolyl, R₂ is halogen, and R₃ is carboxyl or a group that is biologically equivalent thereto;
   the active ingredient being administered at a daily dosage of 120 to 300 mg, preferably 150 to 240 mg, more preferably 150 to 180 mg, and
   the TKI combination drug being orally administered to a chronic myeloid leukemia patient (CML patient) on TKI treatment.
(B) The TKI combination drug according to (A), wherein the compound is a compound of Formula (1) wherein R₁ is quinolin-8-yl and R₃ is carboxyl.
(C) The TKI combination drug according to (A) or (B), which is repeatedly administered to a CML patient orally once daily for 12 weeks or more, preferably 24 weeks or more, more preferably 36 weeks or more.
(D) The TKI combination drug according to any one of (A) to (C), wherein the TKI is imatinib, nilotinib, dasatinib, or bosutinib.
(E) The TKI combination drug according to any one of (A) to (D), wherein the CML patient is a patient undergoing TKI treatment and having a BCR-ABL^{IS} of more than 0.0032% and not exceeding 0.1%.
(F) The TKI combination drug according to any one of (A) to (E), wherein the TKI combination drug is a drug that aids or reinforces the therapeutic effect of the TKI on CML patients (an enhancer for the CML therapeutic effect of the TKI).
(G) A CML treatment method, preferably a curative therapy for CML, comprising administering a pharmaceutical composition comprising present compound (1) or a pharmaceutically acceptable salt thereof as an active ingredient to a CML patient on TKI treatment,
   the pharmaceutical composition being orally administered at a rate such that the daily dosage for the patient is 120 to 300 mg, preferably 150 to 240 mg, more preferably 150 to 180 mg, in terms of the amount of present compound (1).
(H) A pharmaceutical composition to be used in combination with a TKI, for use in the improvement of a clinical condition of a CML patient on TKI treatment, preferably for a curative therapy for CML,
   the pharmaceutical composition comprising present compound (1) or a pharmaceutically acceptable salt thereof as an active ingredient, and
   the method comprising orally administering the pharmaceutical composition to the patient at a rate such that the daily dosage of compound (1) is 120 to 300 mg, preferably 150 to 240 mg, more preferably 150 to 180 mg.
(I) Use of present compound (1) or a pharmaceutically acceptable salt thereof for producing a TKI combination drug to be used in combination with a TKI, preferably an enhancer for the CML therapeutic effect of the TKI,
   the TKI combination drug being orally administered at a daily rate of 120 to 300 mg, preferably 150 to 240 mg, more preferably 150 to 180 mg, to a CML patient on TKI treatment.

The background of the demand for the CML curative therapy and the usefulness of present compound (1) are explained below.

CML is caused by CML stem cells carrying the BCR-ABL gene (hereinafter simply referred to as "CML stem cells") resulting from gene mutations in hematopoietic stem cells. CML stem cells are the source of BCR-ABL-positive CML cells (hereafter, "CML cells") that continue to proliferate due to the tyrosine kinase activity of BCR-ABL. CML treatment is mainly TKI treatment using TKIs such as imatinib. The primary therapeutic goal of TKIs is to prevent blast crisis (hereinafter, "BC") of CML. If the BCR-ABL clones shrinks to at least the major molecular response (hereinafter, "MMR"), i.e., 0.1% or less according to the International Standards (hereinafter, "IS") for BCR-ABL, it rarely leads to BC as long as TKI treatment is continued. On the other hand, TKIs inhibit BCR-ABL tyrosine kinase, suppress the proliferation of CML cells, and show cytotoxic effects; however, it is known that CML stem cells, which hide in the bone marrow niche and are considered quiescent, are insensitive to TKIs. According to several mathematical models, it would take more than 30 years of continuous treatment to completely deplete CML cells, and realistically, treatment interruption would be impossible. Therefore, TKIs need to be taken throughout life, leading to side effects and medical cost burden. However, several prospective studies have revealed that some CML patients on long-term TKI administration become free of molecular genetic relapse, i.e., treatment-free remission (hereinafter, "TFR"), after discontinuation of TKI treatment. As a result of examining which CML patients could discontinue TKI treatment, it was found that at least a clonal reduction of 0.0032% or less in deep molecular response (DMR) BCR-ABL^{IS}, which is a state of molecular remission deeper than MMR, sustained for a certain period of time was a prerequisite for discontinuing TKI treatment. On the other hand, when TKI treatment was discontinued at the MMR level for some inevitable reasons, molecular relapse was always observed. That is, it has become clear that DMR is the first milestone to achieve TFR, and that TFR is impossible without DMR. The NCCN's CML guidelines, revised in 2017, set a minimum requirement for patients and physicians who discontinue TKI treatment based on their own judgment, outside of clinical studies, that TKI treatment can be discontinued only if the patient has been on TKIs for at least 3 years and has maintained DMR for at least 2 of those 3 years.

However, in a prospective clinical study of imatinib development, the achievement rate of DMR tends to be accumulated time-dependently; however, only a handful of patients (5% per year) achieve DMR. Even with the second-generation TKIs with more potent BCR-ABL inhibitory activity, DMR was about 10% cumulative per year over the longest observation, which was 5 years. That is, according to the NCCN guidelines, the percentage of patients who could maintain DMR for more than 2 years after 3 years of treatment with the first-generation imatinib is about 5% of all CML cases, and the percentage of patients who could maintain DMR for more than 2 years after 3 years of treatment with the second-generation TKIs is only 10% of all CML cases.

Present compound (1) is the lead compound of a PAI-1 inhibitor produced by SBDD technology based on the X-ray crystallographic information of human PAI-1. As described in the present specification, the present compound exhibits an effective fibrinolytic enhancing effect at a safe dosage. As shown in Experimental Examples 9 and 10, it was confirmed that repeated daily administration of the prescribed dose in combination with TKI treatment to CML patients on TKI treatment enhanced the therapeutic effect of TKIs while ensuring safety for the CML patients. This indicates that complete cure of CML (maintenance of complete remission) is possible by combining the present compound to TKI treatment.

### Industrial Applicability

As described above, the present compound has been confirmed to be safe for repeated oral administration, and has an inhibitory effect on intrapulmonary microthrombosis, a suppressive effect on pulmonary fibrosis, an emphysema suppressive effect, an inflammation-improving effect (including for cytokine syndrome, particularly severe cytokine storm), and the like. From this, the present compound is useful as a practical medicine for preventing the aggravation of respiratory inflammation associated with coronavirus infections, including COVID-19, and progression to ARDS.

## Claims

1. A drug for enhancing the fibrinolytic system, comprising a compound represented by Formula (1) or a pharmaceutically acceptable salt thereof as an active ingredient: 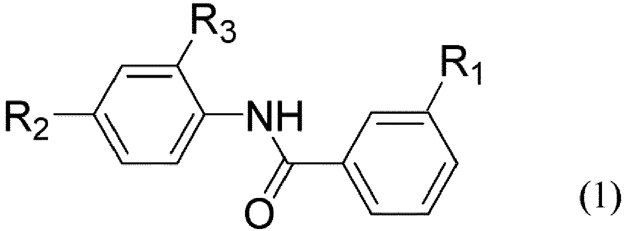 wherein R₁ is quinolyl, R₂ is halogen, and R₃ is carboxyl or a group that is biologically equivalent thereto;
the active ingredient being administered at a daily dosage of 120 to 300 mg, and
the drug being orally administered to a patient with a clinical condition that is expected to be improved by fibrinolytic enhancement.

2. The drug for enhancing the fibrinolytic system according to claim 1, wherein the compound is a compound of Formula (1) wherein R₁ is quinolin-8-yl and R₃ is carboxyl.

3. The drug for enhancing the fibrinolytic system according to claim 1 or 2, which is repeatedly administered orally once daily for at least 7 days.

4. The drug for enhancing the fibrinolytic system according to any one of claims 1 to 3, wherein the patient is a patient having thrombosis, fibrosis, emphysema, and/or inflammation in a respiratory organ.

5. The drug for enhancing the fibrinolytic system according to any one of claims 1 to 4, wherein the patient is a pneumonia patient with a coronavirus infection.

6. A method for improving a clinical condition that is expected to be improved by fibrinolytic enhancement or suppressing the aggravation of the clinical condition, the method comprising orally administering a pharmaceutical composition comprising a compound represented by Formula (1) or a pharmaceutically acceptable salt thereof as an active ingredient: 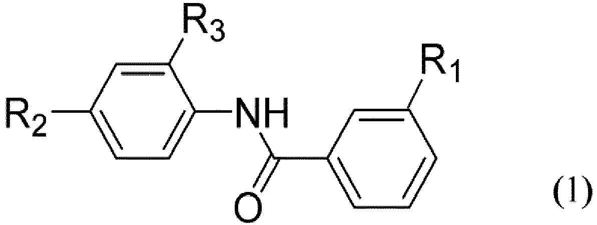 wherein R₁ is quinolyl, R₂ is halogen, and R₃ is carboxyl or a group that is biologically equivalent thereto; to a patient with the clinical condition, preferably an adult patient aged 15 years or older,
the pharmaceutical composition being orally administered at a rate such that the daily dosage for the patient is 120 to 300 mg, preferably 120 to 240 mg, in terms of the amount of compound (1).

7. The method according to claim 6, wherein the pharmaceutical composition is repeatedly administered orally once daily for at least 7 days.

8. The method according to claim 6 or 7, wherein the patient is a patient having thrombosis, fibrosis, emphysema, and/or inflammation in a respiratory organ.

9. The method according to any one of claims 6 to 8, wherein the patient is a pneumonia patient with a coronavirus infection, and the method inhibits progression to acute respiratory distress syndrome.

10. A pharmaceutical composition for use in a method for improving a clinical condition that is expected to be improved by fibrinolytic enhancement or suppressing the aggravation of the clinical condition for a patient with the clinical condition, preferably an adult patient aged 15 years or older,
the pharmaceutical composition comprising a compound represented by Formula (1) or a pharmaceutically acceptable salt thereof as an active ingredient: 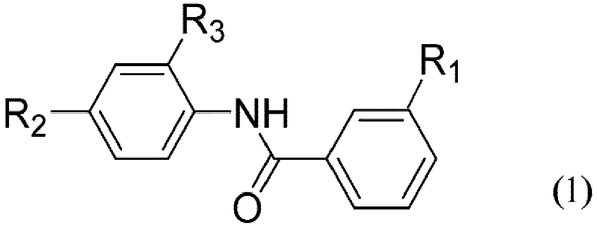 wherein R₁ is quinolyl, R₂ is halogen, and R₃ is carboxyl or a group that is biologically equivalent thereto;
the method comprising orally administering the pharmaceutical composition to the patient at a rate such that the daily dosage of compound (1) is 120 to 300 mg, preferably 120 to 240 mg.

11. The pharmaceutical composition according to claim 10, wherein the method comprises repeatedly administering orally the pharmaceutical composition to the patient once daily for at least 7 days.

12. The pharmaceutical composition according to claim 10 or 11, wherein the patient is a patient having thrombosis, fibrosis, emphysema, and/or inflammation in a respiratory organ, preferably a pneumonia patient with a coronavirus infection.

13. The pharmaceutical composition according to claim 12, which inhibits the progression of the pneumonia patient with a coronavirus infection to acute respiratory distress syndrome.

14. Use of a compound represented by Formula (1) or a pharmaceutically acceptable salt thereof: 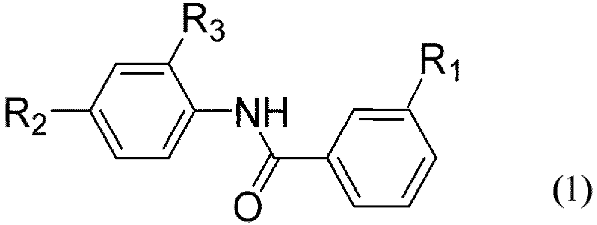 wherein R₁ is quinolyl, R₂ is halogen, and R₃ is carboxyl or a group that is biologically equivalent thereto; for producing a drug for enhancing the fibrinolytic system,
the drug for enhancing the fibrinolytic system being orally administered at a daily rate of 120 to 300 mg, preferably 120 to 240 mg, to a patient with a clinical condition that is expected to be improved by fibrinolytic enhancement.

15. The use according to claim 14, wherein the patient is a patient having thrombosis, fibrosis, emphysema, and/or inflammation in a respiratory organ, preferably a pneumonia patient with a coronavirus infection.
